# FASCICULE DE BREVET EUROPEEN

(11) **EP 4 398 796 B1**
(45) Date de publication et mention de la délivrance du brevet: **26.11.2025**
(21) Numéro de dépôt: 22776920.5
(22) Date de dépôt: 07.09.2022
(51) Int. Cl.: A61B 5/20, A61B 5/145, A61B 5/00, A61B 10/00, G01N 33/493

(54) **STATION POUR DISPOSITIF D'ANALYSE D'URINE ET DISPOSITIF D'ANALYSE D'URINE**
STATION FÜR EINE URINANALYSEVORRICHTUNG UND URINANALYSEVORRICHTUNG
STATION FOR A URINE ANALYSIS DEVICE AND URINE ANALYSIS DEVICE

(30) Priorité: 08.09.2021 FR 2109383
(43) Date de publication de la demande: 17.07.2024
(73) Titulaire: Withings, 92130 Issy-Les-Moulineaux (FR)
(72) Inventeur: TINÉ, Jorys, 92130 Issy-les-Moulineaux (FR); TUCOULAT, Benoît, 92130 Issy-les-Moulineaux (FR); KIRAT, Marine, 92130 Issy-les-Moulineaux (FR); LOY, Jonas, 92130 Issy-les-Moulineaux (FR); DEWAVRIN, Julius, 92130 Issy-les-Moulineaux (FR); BARAKAT, Christelle, 92130 Issy-les-Moulineaux (FR)
(74) Mandataire: Withings IP
(86) Numéro de dépôt international: PCT/EP2022/074830
(87) Numéro de publication internationale: WO 2023/036805

(56) Documents cités:
- US-A1- 2007 020 143
- US-A1- 2013 324 807
- US-A1- 2017 284 925
- US-B1- 9 810 686

## Description

### Domaine technique

La présente divulgation relève du domaine des dispositifs d'analyse d'urine destinés à être positionnés à l'intérieur de toilettes, partiellement ou complètement. La présente divulgation vise également une méthode pour analyser l'urine étant reçue dans des toilettes.

### Technique antérieure

De nombreux paramètres biologiques sont reflétés dans l'urine d'un individu. A partir d'un échantillon d'urine, il est par exemple possible de détecter des problèmes de santé tels qu'une infection urinaire, le diabète ou une insuffisance rénale. L'échantillon d'urine peut également refléter la qualité d'une alimentation, identifier une période de fécondité ou une grossesse et détecter une prise de drogues ou de tabac. Il est alors intéressant de surveiller divers paramètres biologiques périodiquement.

Il est connu de proposer des dispositifs installés dans des toilettes ayant une fonction d'analyse d'urine. Ces dispositifs sont capables d'effectuer des prélèvements d'échantillons d'urine depuis les toilettes et de les analyser afin de déterminer le niveau d'un paramètre biologique.

On connait du document US20180188231 un dispositif d'analyse d'urine à fixer sur un rebord de toilettes. Ce dispositif permet de réaliser une analyse avec un transistor à effet de champ.

Il est proposé dans les documents US20170284925 et US10383606 des dispositifs comportant des bandelettes de test défilant devant une section d'analyse.

Cependant, de tels dispositifs sont volumineux. Notamment, ils nécessitent une zone de stockage de bandelettes de test neuves et de bandelettes de tests usagées. Ces dispositifs doivent alors être positionnés en grande partie à l'extérieur des toilettes ou doivent être intégrés à celles-ci.

Par ailleurs, de tels dispositifs ne sont pas flexibles. Il apparait particulièrement difficile de recharger des bandelettes de test neuves et de retirer des bandelettes usagées. Il apparait également difficile de réaliser des analyses nécessitant plusieurs types de bandelettes.

Le document US2013/0324807 décrit un magazine analytique circulaire et rotatif qui comprend une pluralité d'aides analytiques accommodés dans des chambres. Le document US9810686 décrit une cassette d'analyse d'urine insérée dans des toilettes. Le document US2007/0020143 décrit une bande pour analyser des fluides corporels.

Il existe donc un besoin pour un dispositif d'analyse d'urine ne présentant pas les inconvénients de l'art antérieur.

### Résumé

La présente description vise à proposer une ou plusieurs solutions résolvant au moins certains des inconvénients précités. En particulier, le déposant a développé un nouveau dispositif comprenant une station et une cartouche (aussi appelé support rotatif), décrit dans le document PCT/EP2021/055302. La présente demande vise à proposer des améliorations du dispositif et de la station de ce document L'invention est définie dans les revendications

Il est ici proposé une station d'analyse d'urine, configuré pour coopérer avec une cartouche, de façon à former un dispositif d'analyse d'urine. Il est aussi ici proposé différentes méthodes d'utilisation du dispositif d'analyse d'urine.

La station comprend un boitier qui est destiné à être positionné à l'intérieur de toilettes, c'est-à-dire disposé à l'intérieur de la cuvette. Le boitier comprend l'électronique nécessaire au fonctionnement de la station et du dispositif. Le corps principal est positionné pour recevoir un jet d'urine lorsque l'utilisateur urine. La station peut comprendre un logement annulaire, dans le boitier, c'est-à-dire un espace vide de forme annulaire, qui s'étend autour d'un axe de rotation. La station peut comprendre en outre un injecteur d'urine, positionné dans le boitier, en particulier positionnée radialement à l'intérieur du logement annulaire. Le logement annulaire est configuré pour accueillir, entièrement ou partiellement, une cartouche, qui est mobile en rotation dans la station, autour de l'axe de rotation. La cartouche comprend typiquement un support rotatif qui loge une pluralité de support de test (par exemple des bandelettes de test) disposées le long d'un arc de cercle. Les supports de test sont attachés au support rotatif et restent attaché à ce dernier lors de l'utilisation du dispositif d'urine. Dit autrement, tous les supports de test, à tout instant, subissent le même mouvement de rotation lorsque la cartouche est entrainée en rotation (par exemple, dans le cas de bandelettes de test, pas d'enroulement ou de déroulement d'une pellicule de bandelettes autour d'un enrouleur et/ou d'un dérouleur). Lors de la rotation de la cartouche dans la station, les supports de test peuvent être ainsi sélectivement disposés devant l'injecteur d'urine qui peut alors injecter un volume contrôlé d'urine. Pour insérer la cartouche dans le corps principal, ce dernier peut comprendre un capot amovible afin de pouvoir accéder au logement annulaire. Le corps principal peut aussi comprendre un analyseur configuré pour analyser un support de test. De la même façon que précédemment, lors de la rotation de la cartouche dans la station, les supports de test peuvent être ainsi sélectivement disposées devant l'analyseur qui peut alors obtenir des données sur le support de test qui se trouve en regard de ce dernier. L'analyse est typiquement une analyse optique, comme une analyse colorimétrique. Pour positionner correctement le support de test vis-à-vis de l'injecteur et/ou de l'analyseur, la station peut comprendre un capteur de position, qui permet d'obtenir une position, au moins localement, de chaque support de test par rapport au boitier (et donc par rapport au logement annulaire). En particulier, au moins deux parmi l'injecteur, l'analyseur et le capteur de position, sont positionnées le long d'une direction radiale autour de l'axe de rotation au même endroit, pour travailler sur un même support de test sans qu'une rotation de la cartouche ne soit nécessaire entre l'obtention de la position et l'injection, ou entre l'obtention de la position et la mesure, ou entre l'injection et la mesure. De la sorte, l'injection et l'analyse sont optimisées.

Le support de test comprend un réactif apte à réagir une fois au contact de l'urine. Le réactif peut être un réactif sec. Dans un mode de réalisation, le support de test est une bandelette de test mais d'autres supports peuvent être implémentés.

Ici le terme « radialement » signifie le long d'une direction radiale. Une telle direction radiale est généralement définie comme une direction perpendiculaire à l'axe de rotation (A) et passant par l'axe de rotation (A).

Dans un mode de réalisation, l'injecteur est mobile par rapport au corps principal. En particulier, on définit une position d'injection, dans laquelle l'injecteur peut déposer quelques gouttes d'urine sur un support de test, une position de purge, dans laquelle l'injecteur peut évacuer de l'urine vers une décharge et une position de retrait, dans laquelle l'injecteur n'est pas configuré pour être actif. Pendant les rotations de la cartouche, l'injecteur est typiquement en position de retrait. Dans un mode de réalisation, le mouvement effectué par l'injecteur est une translation.

En particulier, l'injecteur est positionné radialement interne au logement annulaire, de sorte que le logement annulaire entoure l'injecteur. A l'exception de l'extrémité distale, qui pénètre dans le logement annulaire du fait du déplacement de l'injecteur, le reste de l'injecteur demeure radialement interne au logement annulaire. Dans certaines positions (position de purge et position d'injection), tout l'injecteur reste radialement interne au logement annulaire. En position de purge, l'extrémité distale se trouve même radialement externe au logement annulaire.

La station peut aussi comprendre aussi une unité électronique de contrôle ECU et un module de communication (typiquement sans fil), pour communiquer bidirectionnellement avec un terminal mobile et/ou un serveur distant. Une batterie dans le boitier permet d'alimenter en énergie tous les composants

La présente description couvre plusieurs aspects. Certains d'entre eux vont être mis en avant dans les paragraphes suivants, mais la description ne se limite pas à ceux-ci.

Selon l'invention, dans un aspect (appelé « injecteur courbé »), il est proposé une station pour dispositif d'analyse d'urine, la station comprenant :
- un boitier, destiné à être positionné à l'intérieur de toilettes,
- un logement annulaire, autour d'un axe de rotation, dans le boitier, le logement annulaire étant configuré pour recevoir au moins partiellement une cartouche montée à rotation autour de l'axe de rotation dans la station et comprenant une pluralité de supports de test de test,
- un injecteur, positionné dans le boitier, et configuré pour injecter un volume contrôlé d'urine sur au moins un support de test, l'injecteur étant monté mobile en translation par rapport au boitier.

L'injecteur comprend une extrémité distale d'injection dont le déplacement en translation se fait selon une direction radiale ou sensiblement radiale par rapport à l'axe de rotation.

« Sensiblement radiale » peut signifier moins de 5° de part et d'autre de la direction radiale, voire moins de 2°. Plus on est proche d'une direction radiale, plus on gagne en marge au niveau du déplacement de la cartouche.

L'injecteur est positionné radialement interne au logement annulaire, de sorte que le logement annulaire entoure l'injecteur. A l'exception de l'extrémité distale, qui pénètre dans le logement annulaire du fait du déplacement radial, le reste de l'injecteur demeure radialement interne au logement annulaire. Dans certaines positions (position de retrait notamment), tout l'injecteur reste radialement interne au logement annulaire. En position de purge, l'extrémité distale se trouve même radialement externe au logement annulaire.

L'injecteur peut être entrainé en translation au niveau d'un premier axe, tandis que l'extrémité distale d'injection se déplace en translation au niveau d'un deuxième axe, dit axe d'injection. Le premier axe de translation n'est alors pas confondu avec le deuxième axe, une fois ces derniers projetés dans un plan orthogonal à l'axe de rotation. Par « au niveau », il est signifié que le premier axe porte l'axe d'actionneur de déplacement alors que deuxième axe porte l'axe d'injection, les deux axes étant parallèles mais décalés.

L'injecteur peut être est courbé. En d'autres termes, l'injecteur comprend, une fois projeté dans un plan orthogonal à l'axe de rotation, une forme courbée. On entend ici que le canal de l'injecteur qui conduit le fluide est courbe.

Le boitier peut comprendre un coupleur mécanique central, sur l'axe de rotation, apte à coopérer avec la cartouche pour la mise en rotation de cette dernière. Le deuxième axe de translation peut alors traverser ledit coupleur mécanique et le premier axe de translation peut être déporté par rapport au coupleur mécanique. La forme courbée de l'injecteur contourne le coupleur mécanique. Typiquement, le deuxième axe de translation est séparé du premier axe d'une distance de déport déterminé (par exemple au moins 5mm et par exemple moins de 15mm voire 10mm).

L'injecteur peut comprend une extrémité proximale, opposée à l'extrémité distale et configuré pour recevoir de l'urine. L'extrémité distale et l'extrémité proximale sont alors reliées entre elles par une portion intermédiaire comprenant deux virages successifs inversés. En particulier, le virage le plus proche de l'extrémité distal (virage distal) a un rayon de courbure plus faible que le virage le plus proche de l'extrémité proximale.

L'injecteur peut être rigide.

La station peut comprendre un actionneur de déplacement, configuré pour déplacer l'injecteur, notamment en translation.

Pour le déplacement en translation de l'injecteur, ce dernier peut être solidaire d'un écrou apte à recevoir une tige filetée entrainée en rotation par l'actionneur de déplacement. L'injecteur

(604) peut en outre être monté sur un chariot et le chariot peut reprendre les efforts transmis par l'actionneur de déplacement. Par exemple, le chariot est entrainé en translation au niveau du premier axe. L'écrou peut être monté sur le chariot.

Le chariot peut comprend un guide configuré pour coopérer avec un guide du boitier pour guider ledit chariot en translation. On fournit une liaison glissière qui permet de compenser le couple parasite généré par le déport entre l'axe d'injection et l'axe d'actionneur de déplacement.

Le boitier peut comprend un collecteur d'urine, ledit collecteur étant fluidiquement relié à l'injecteur, en particulier via l'extrémité proximale. Le boitier peut comprendre une unité électronique de contrôle, l'unité électronique de contrôle étant donc dans le boitier.

La station peut comprendre un analyseur (par exemple un analyseur optique), positionné au moins partiellement radialement à l'extérieur du logement annulaire et configuré pour analyser optiquement au moins un support de test. L'analyseur peut être positionné en regard de l'extrémité distale de l'injecteur (lorsqu'ils sont projetés dans un plan orthogonal à l'axe de rotation).

Dans un aspect (appelé « capteur de position et injecteur »), il est proposé une station pour dispositif d'analyse d'urine, la station comprenant :
- un boitier, destiné à être positionné à l'intérieur de toilettes,
- un logement annulaire, autour d'un axe de rotation, dans le boitier, le logement annulaire étant configuré pour recevoir au moins partiellement une cartouche montée à rotation autour de l'axe de rotation dans la station et comprenant une pluralité de supports de test,
- un injecteur, positionné dans le boitier, et configuré pour injecter un volume contrôlé d'urine sur un support de test , lorsque la bandelette est positionnée dans une zone d'injection du logement annulaire,
- un capteur de position, configuré pour obtenir la position d'un marqueur associé à un support de test de la cartouche lorsque ledit marqueur est positionné dans la zone d'injection ou dans une zone de proximité de la zone d'injection.

Sous le terme « zone d'injection », on désigne en particulier une plage angulaire dans le repère autour de l'axe de rotation. L'injecteur et le capteur de position sont donc agencés pour interagir avec le même support de test, sans rotation de la cartouche entre l'obtention de la position et l'injection. En particulier, le marqueur peut être un support de test directement.

La zone d'injection peut correspondre à l'angle occupé par une seul support de test (en équivalent fenêtre angulaire). Par exemple, la zone d'injection peut correspondre à une fenêtre angulaire de moins de 5°, voire moins de 2° de part et d'autre de la direction radiale.

La zone de proximité de la zone d'injection peut correspondre à un secteur angulaire de moins de 30° de part et d'autre de la zone d'injection (des bords de la zone d'injection), voire moins 20°, voire moins de 10°, voire moins de 5°.

L'injecteur peut comprendre une extrémité distale d'injection et le capteur de position est radialement aligné (par projection dans un plan orthogonal à l'axe de rotation) avec l'extrémité distale de l'injecteur. L'injecteur et le capteur de position peuvent être décalés le long de l'axe de rotation (par projection sur l'axe de rotation).

Le capteur de position peut être positionné au moins partiellement radialement à l'extérieur du logement annulaire.

Le boitier peut comprend un analyseur configuré pour analyser au moins un support de test de test. En particulier, l'analyseur est un analyseur optique.

L'injecteur peut être configuré pour injecter dans une zone d'injection du logement annulaire et l'analyseur est configuré pour mesurer des données dans une zone d'analyse, la zone d'injection et la zone d'analyse étant confondues. L'analyseur peut être positionné radialement en regard de l'extrémité distale de l'injecteur ( lorsqu'ils sont projetés dans un plan orthogonal à l'axe de rotation). Le capteur de position peut être l'analyseur (même matériel mais fonction différente). L'injecteur peut être monté mobile en translation par rapport au boitier selon une direction de translation, pour se rapprocher et/ou traverser le logement annulaire.

Le capteur de position peut alternativement ou complémentairement comprendre un suiveur mécanique, comme un suiveur de came, (qui coopère avec la cartouche) ou un module électromagnétique (qui coopère avec un élément magnétique de la cartouche).

Dans un aspect (appelé « analyseur et injecteur »), il est proposé une station pour dispositif d'analyse d'urine, la station comprenant :
- un boitier, destiné à être positionné à l'intérieur de toilettes,
- un logement annulaire autour d'un axe de rotation, positionné dans le boitier, le logement annulaire étant configuré pour recevoir au moins partiellement une cartouche montée à rotation autour de l'axe de rotation dans la station et comprenant une pluralité de supports de test,
- un analyseur, monté dans le boitier, et configuré pour obtenir une information relative à le support de test après injection d'urine, lorsque le support de test est dans une zone d'analyse du logement annulaire,
- un injecteur, monté dans le boitier, et configuré pour injecter un volume contrôlé d'urine sur un support de test, lorsque le support de test est dans une zone d'injection du logement annulaire.

La zone d'analyse et la zone d'injection sont confondues. De la sorte, il n'y a pas besoin de faire tourner la cartouche entre l'injection et la mesure par l'analyseur. L'analyseur et l'injecteur sont donc situés angulairement au même endroit autour de l'axe de rotation (décalage possible le long de l'axe de rotation).

La station comprend en outre un capteur de position, configuré pour obtenir la position d'un marqueur associé à un support de test de la cartouche, lorsque ledit marqueur est positionné dans la zone d'analyse ou dans une zone de proximité de la zone d'analyse. La zone de proximité de la zone d'analyse peut correspondre à un secteur angulaire de moins 30° de part et d'autre de la zone d'injection, voire 20°, voire 10°, voire 5°. La zone d'analyse peut quant à elle correspondre à un secteur angulaire inférieure ou égal à l'angle occupée par un seule support de test.

L'injecteur peut être monté mobile en translation dans le boitier.

La station peut comprendre une mémoire et un processeur, ladite mémoire comprenant des instructions configurées pour mettre en oeuvre les étapes suivantes :
- (E3) injection d'urine sur un support de test par l'injecteur,
- (E4) mesure sur le support de test par l'analyseur.

En particulier les instructions ne comprennent pas de mise en rotation de la cartouche entre l'étape d'injection (E3) et l'étape d'analyse (E4). Les instructions peuvent comprendre que l'étape de mesure (E4) commence avant la fin de l'étape d'injection (E3), par exemple deux secondes avant la fin ou dès l'injection de la première goutte d'urine, voire avant l'injection de la première goutte d'urine. Cela permet d'obtenir des mesures relatives à la cinétique des réactions ayant lieu sur le support de test mis au contact de l'urine.

Il est aussi proposé une méthode d'analyse d'urine exploitant le dispositif d'analyse d'urine tel que décrit précédemment, comprenant les étapes suivantes :
- (E3) injection d'urine sur le support de test,
- (E4) mesure sur le support de test par l'analyseur,
dans lequel la cartouche n'est pas mise en rotation entre l'étape d'injection et l'étape d'analyse.

Dans un aspect (appelé « mesure directe de la position du support de test »), il est proposé une station pour dispositif d'analyse d'urine, la station comprenant :
- un boitier, destiné à être positionné à l'intérieur de toilettes,
- un logement, dans le boitier, configuré pour recevoir au moins partiellement une cartouche comprenant une pluralité de supports de test de test, la cartouche étant montée mobile à l'intérieur du logement,
- un capteur de position, configuré pour mesurer directement la position d'un support de test de la cartouche dans le logement annulaire, c'est-à-dire par rapport au boitier.

En particulier, le capteur de position ne mesure pas la position de la cartouche, plus particulièrement pas la position du support rotatif de la cartouche ou du séparateur. Ainsi, tout imprécision du positionnement support de test sur le support rotatif est surmonté en récupérant directement la position du support de test dans le logement annulaire.

Le capteur de position peut comprendre une source de lumière et un capteur optique, la source de lumière étant configurée pour émettre une lumière en direction d'un support de test et le capteur optique étant configuré pour recevoir la lumière.

La station peut comprendre un analyseur, l'analyseur travaillant dans une zone d'analyse du logement, et le capteur de position est configuré pour mesurer directement la position d'un support de test se trouvant dans la zone d'analyse. En particulier, l'analyseur peut se trouver angulairement au même emplacement que le capteur optique de position (avec ou sans décalage le long de l'axe de rotation A). La zone d'analyse peut quant à elle correspondre à un secteur angulaire inférieure ou égal à l'angle occupée par un seul support de test.

L'analyseur peut être le capteur de position. En d'autres termes, le matériel formant l'analyseur peut être utilisé comme capteur de position.

Le logement peut être un logement annulaire, autour d'un axe de rotation, dans le boitier, le logement annulaire étant configuré pour recevoir au moins partiellement une cartouche montée à rotation autour de l'axe de rotation dans la station.

Le capteur de position peut voir défiler des supports de test lorsque la cartouche est mise en mouvement, et est apte à recevoir un signal qui varie en fonction de la présence ou non d'un support de test en regard du capteur de position. La station peut comprendre en outre une unité électronique de contrôle avec un processeur et une mémoire. L'unité électronique de contrôle peut être dans le boitier. La mémoire peut comprendre des instructions qui, lorsqu'exécutées par le processeur de l'unité électronique de contrôle provoque les étapes suivantes :
- (F1) mise en défilement des supports de test dans un sens,
- (F2) analyse de l'évolution du signal durant le défilement,
- (F3) identification d'un extremum local,
- (F4) en réponse à ladite identification, mise en défilement inverse des supports de test,
- (F5) mise en position du support de test qui a généré le premier extremum.

La mise en position (F5) peut comprendre :
- après la mise en défilement inverse des supports de test (F4), (F51) identification d'une valeur au voisinage de la valeur de l'extremum local,
- (F52) arrêt du défilement inverse des supports de test.

L'identification de l'extremum local (F3) consiste à obtenir à la suite au moins trois valeurs de signal retour, correspondant à trois positions différentes de la cartouche dans la station et à constater que la valeur intermédiaire est la plus élevé, de sorte qu'au moment de l'identification de l'extremum local, le support de test n'est plus en regard du capteur de position.

Lorsque le logement est un logement annulaire, la mise en défilement peut être une mise en rotation de la cartouche.

Le boitier peut comprendre un injecteur, configuré pour injecter de l'urine sur le support de test qui est identifié comme mise en position par le capteur de position.

La station peut comprendre actionneur d'entrainement, par exemple pas-à-pas, pour entrainer en déplacement la cartouche. Une valeur du signal peut être obtenue à chaque pas.

Il est aussi proposé une méthode de positionnement d'un support de test à l'aide du dispositif comprenant une station telle que décrite précédemment et une cartouche. La méthode comprend une étape de mesure de position d'un support de test directement. Plus spécifiquement, la méthode comprend les étapes suivantes :
- (F1) mise en défilement des supports de test dans un sens,
- (F2) analyse de l'évolution du signal durant le défilement,
- (F3) identification d'un extremum local,
- (F4) en réponse à ladite identification, mise en défilement inverse des supports de test,
- (F5) mise en position du support de test qui a généré le premier extremum.

La présente description couvre aussi un dispositif d'analyse d'urine comprenant une station (telle que décrite selon les différents aspects ci-dessus) et une cartouche. La cartouche est alors configurée pour être au moins partiellement reçue dans le logement annulaire du boitier. Chaque support de test est solidaire de la cartouche et est configuré pour pouvoir être sélectivement positionné devant le capteur de position, l'injecteur et l'analyseur. En particulier, le support de test est une bandelette de test. La présente description couvre aussi un kit comprenant cette station et une cartouche ou une pluralité de cartouches (cartouches avec différents types de supports de test).

### Brève description des dessins

D'autres caractéristiques, détails et avantages apparaîtront à la lecture de la description détaillée ci-après, et à l'analyse des dessins annexés, sur lesquels :
**Fig. 1****.**

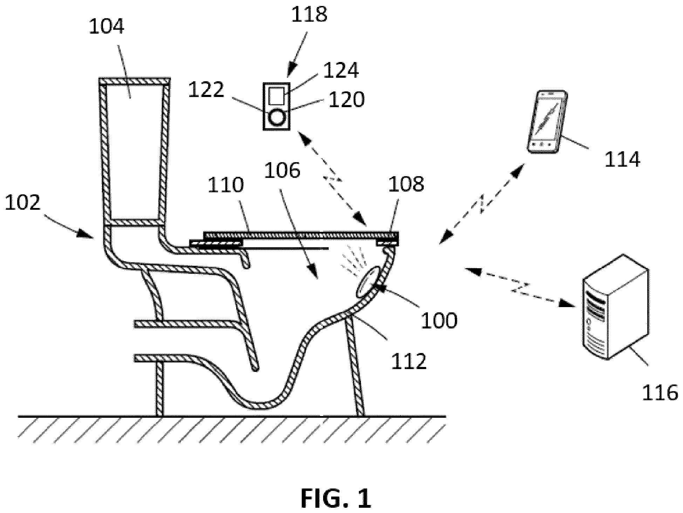

   [Fig. 1] Cette figure représente schématiquement une vue en coupe de toilettes équipées d'un dispositif d'analyse d'urine au sens de l'invention.
**Fig. 2**

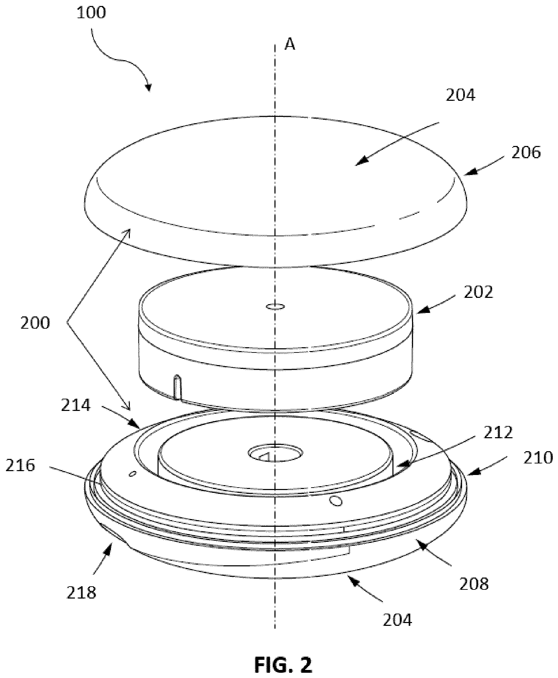

   [Fig. 2] Cette figure représente une vue éclatée d'un mode de réalisation d'un dispositif d'analyse d'urine.
**Fig. 3**

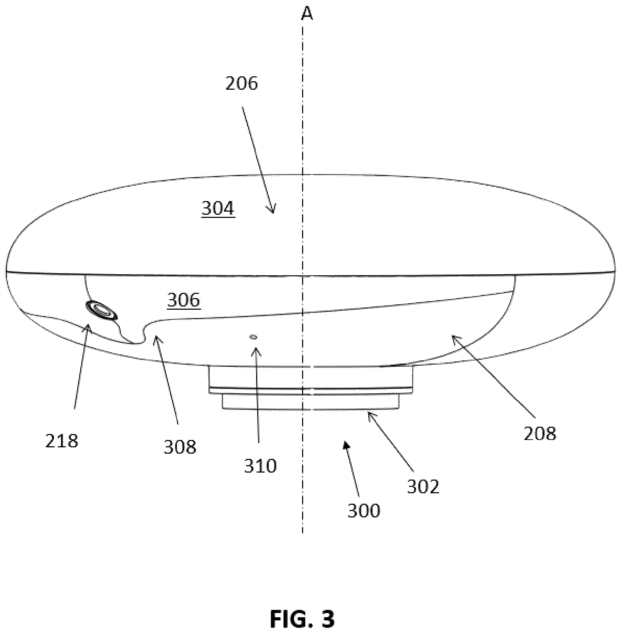

   [Fig. 3] Cette figure représente une vue de côté d'une station ou d'un dispositif d'analyse d'urine selon un mode de réalisation.
**Fig. 4**

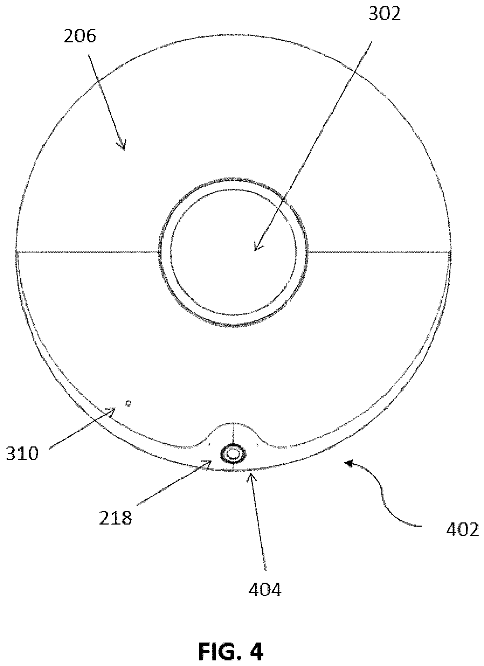

   [Fig. 4] Cette figure représente une vue de la face arrière d'une station ou d'un dispositif d'analyse d'urine selon un mode de réalisation.
**Fig. 5**

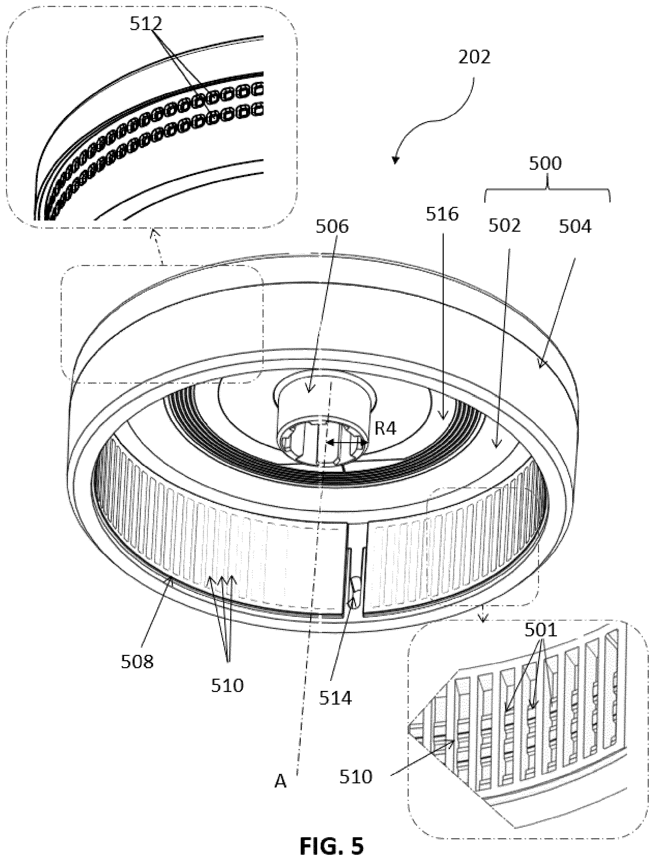

   [Fig. 5] Cette figure représente une cartouche coopérant avec la station pour former un dispositif d'analyse d'urine, avec une vue partielle sans la portion cylindrique pour mieux voir le séparateur.
**Fig. 6**

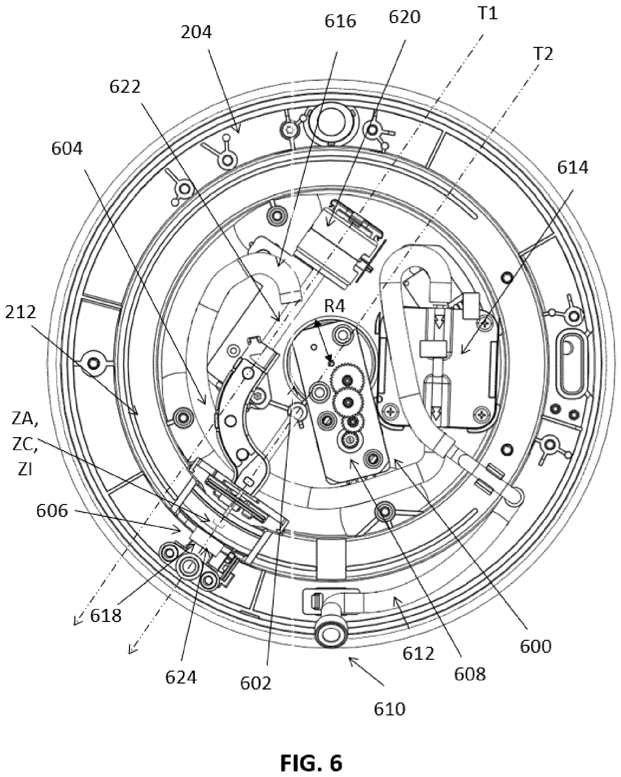

   [Fig. 6] Cette figure présente une vue en coupe de la station selon le plan orthogonal à l'axe de rotation, selon un mode de réalisation de l'injecteur.
**Fig. 7**

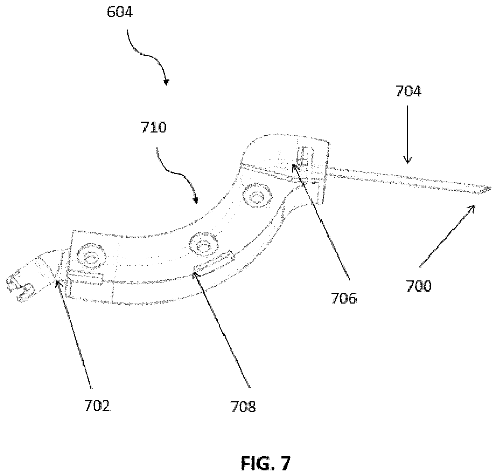

   [Fig. 7] Cette figure présente une vue tridimensionnelle isolée de l'injecteur, selon un mode de réalisation de l'injecteur.
**Fig. 8**

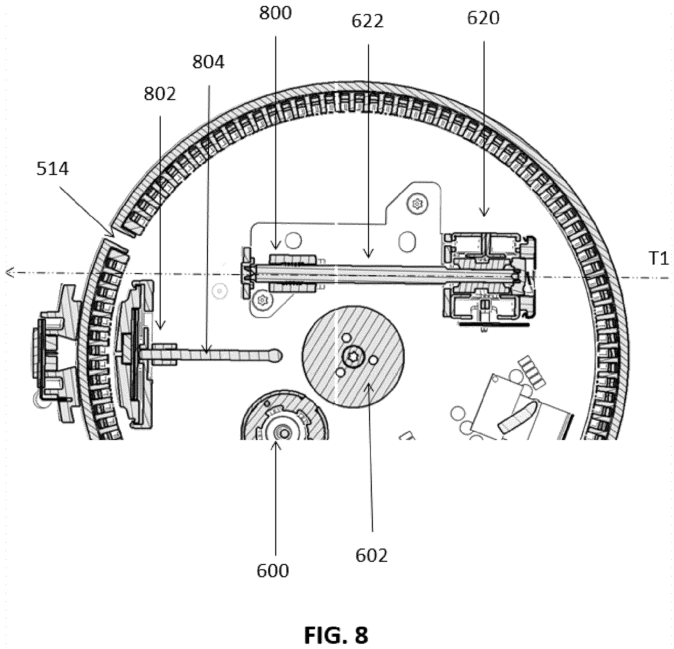

   [Fig. 8] Cette figure présente une vue en coupe du dispositif d'analyse selon un plan orthogonal à l'axe de rotation, selon un mode de réalisation de l'injecteur.
**Fig. 9**

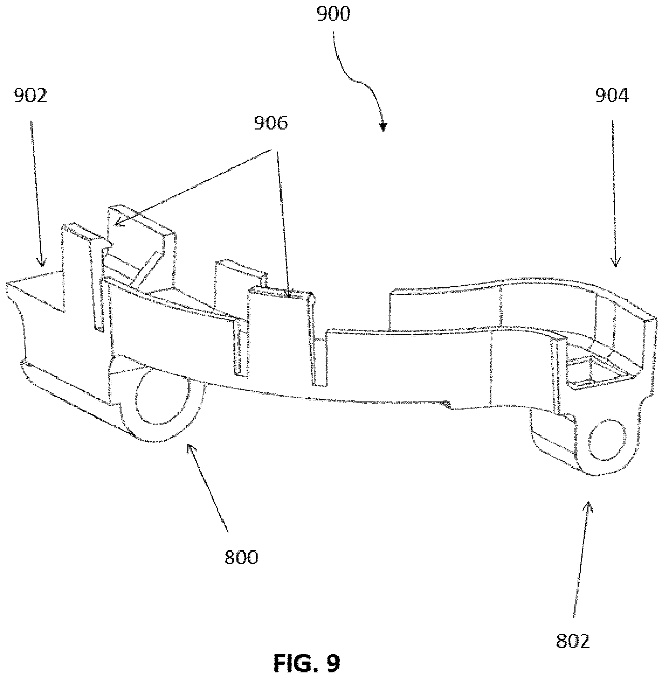

   [Fig. 9] Cette figure présente une vue tridimensionnelle isolée du chariot qui porte l'injecteur, selon un mode de réalisation de l'injecteur.
**Fig. 10**

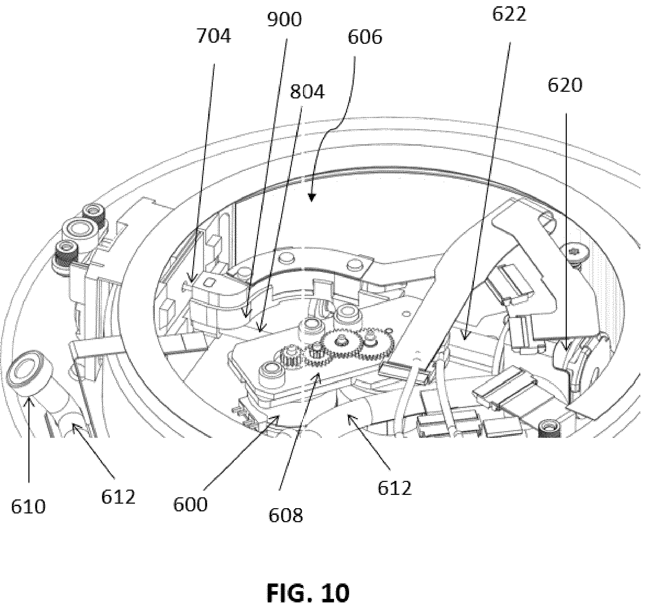

   [Fig. 10] Cette figure présente une vue tridimensionnelle partielle de la station, selon un mode de réalisation.
**Fig. 11**

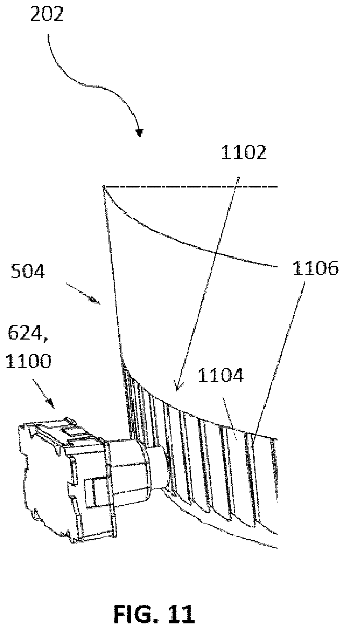

   [Fig. 11] Cette figure présente une vue tridimensionnelle d'un autre mode de réalisation du capteur de position.
**Fig. 12**

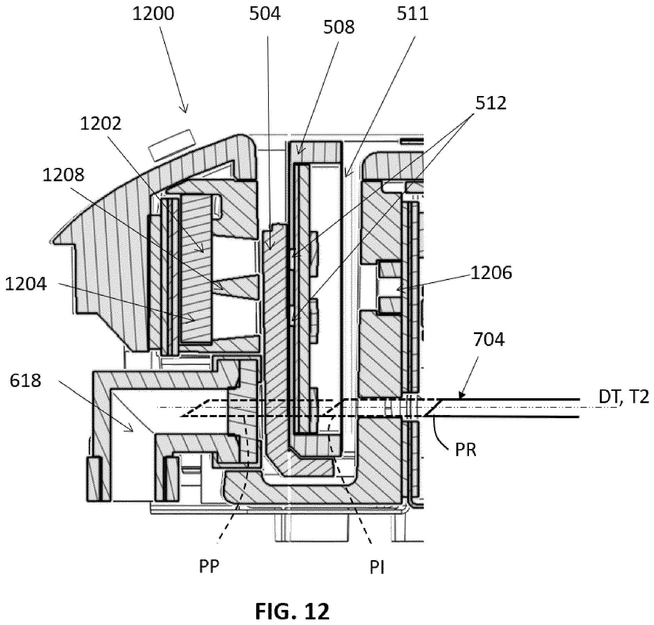

   [Fig. 12] Cette figure présente une vue en coupe, selon un plan passant par une direction radiale et par l'axe de rotation, de la zone d'injection, de la zone d'analyse et la zone de mesure, lorsque les trois zones sont confondues.
**Fig. 13**

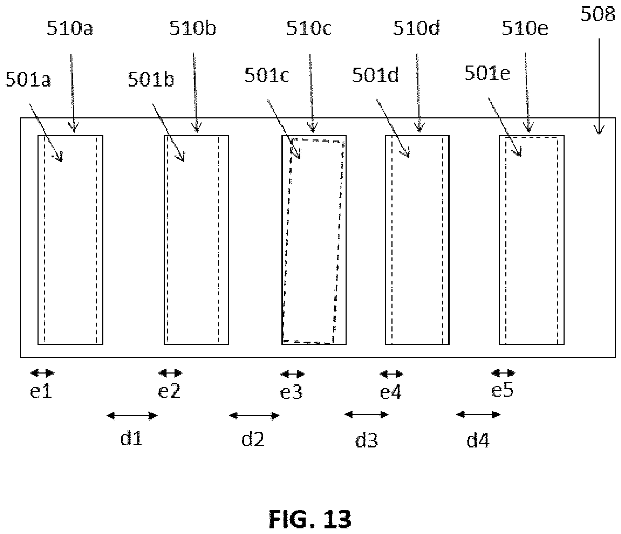

   [Fig. 13] Cette figure présente une vue schématique aplanie d'un séparateur avec des logements et des bandelettes, pour illustrer les irrégularités.
**Fig. 14**

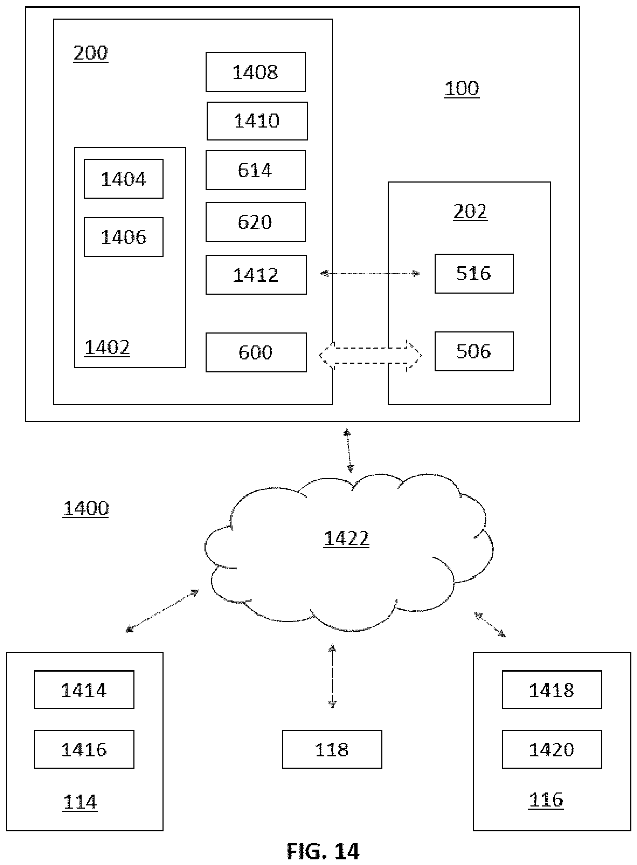

   [Fig. 14] Cette figure présente une vue schématique de certains composants du dispositif d'analyse d'urine et de son environnement.
**Fig. 15**

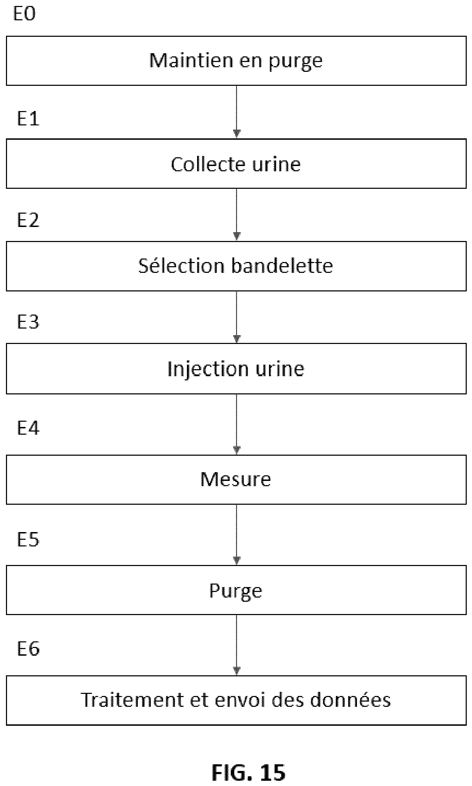

   [Fig. 15] Cette figure présente les étapes d'un procédé d'utilisation du dispositif d'analyse d'urine.
**Fig. 16**

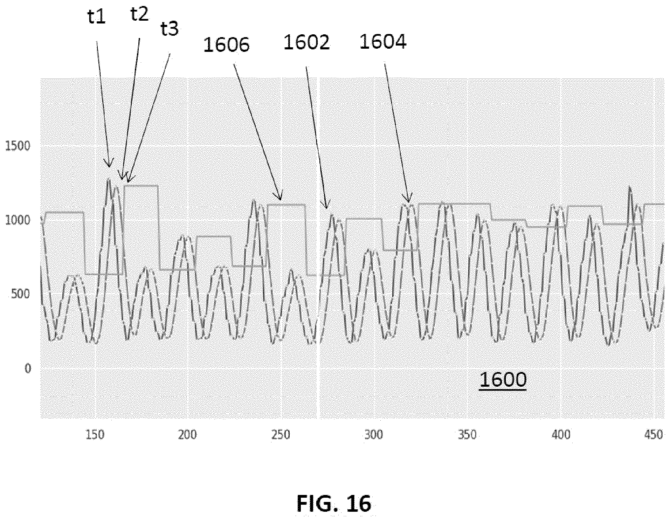

   [Fig. 16] Cette figure présente un graphe avec les données du capteur de position.
**Fig. 17**

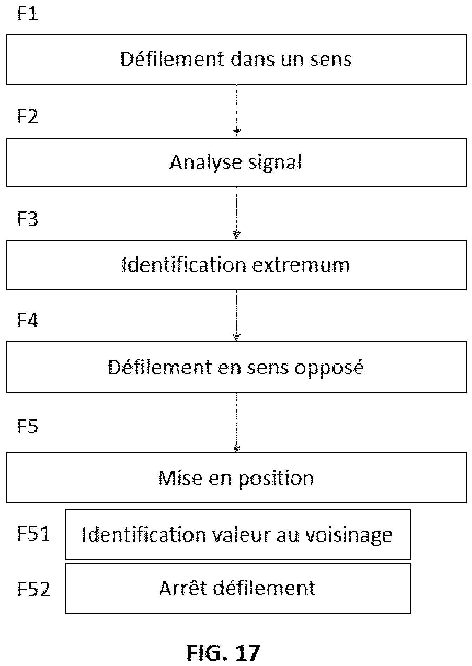

   [Fig. 17] Cette figure présente les étapes d'un procédé de mise en position de la bandelette dans la zone d'injection ou zone d'analyse du dispositif d'analyse d'urine.
**Fig. 18**

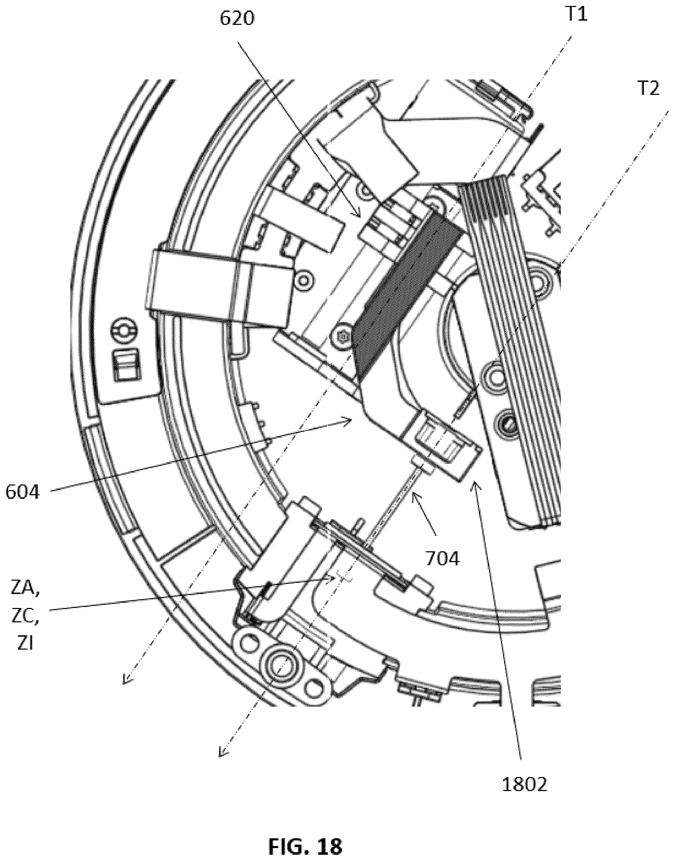

   [Fig. 18] Cette figure présente une vue en coupe de la station selon un autre mode de réalisation, selon le plan orthogonal à l'axe de rotation.

### Description des modes de réalisation

La figure 1 illustre schématiquement un dispositif d'analyse d'urine 100 monté sur des toilettes 102. De manière connue, les toilettes 102 comportent un réservoir d'eau 104, une cuvette 106, un siège 108 et un couvercle 110. Le dispositif d'analyse d'urine 100 est agencé sur une paroi interne 112 de la cuvette 106 des toilettes. Avantageusement, le dispositif d'analyse d'urine 100 est entièrement reçu dans la cuvette des toilettes, ce qui lui permet d'être discret.

Le dispositif d'analyse d'urine 100 peut être positionné sur la trajectoire d'un jet d'urine secrété par un utilisateur. Le dispositif d'analyse d'urine 100 reçoit un jet d'urine lorsqu'un utilisateur urine en position assise dans les toilettes. La position du dispositif d'analyse d'urine est alors adaptée pour tout type d'utilisateur, de sexe masculin ou féminin, quel que soit son âge. L'utilisateur peut alors uriner dans les toilettes sans se préoccuper de la position du dispositif d'analyse d'urine.

Le dispositif d'analyse d'urine 100 peut être également positionné sur la trajectoire d'une chasse d'eau provenant du réservoir 104. Le dispositif d'analyse d'urine 100 peut ainsi être rincé lors de l'actionnement de la chasse d'eau. Le dispositif d'analyse d'urine 100 est hygiénique.

Le dispositif d'analyse d'urine 100 peut communiquer avec un terminal mobile 114 (type smartphone) et/ou un serveur externe 116. Dans un mode de réalisation, le dispositif d'analyse d'urine 100 communique avec le terminal mobile 114 (par exemple directement via Bluetooth comme du Bluetooth Low Energy) et le terminal mobile 114 communique avec le serveur 116 (via une connexion cellulaire ou WiFi). Dans un autre mode de réalisation, le dispositif d'analyse d'urine 100 peut communiquer directement avec le serveur 116 par un réseau cellulaire.

Pour lancer une mesure par le dispositif d'analyse d'urine 100, un activateur externe 118 peut être prévu. L'activateur externe 118 peut comprendre un bouton 120 et/ou un capteur biométrique 122. Un écran 124 peut être installé sur l'activateur externe 118 pour afficher les données obtenues par le dispositif d'analyse d'urine 100. Ce dernier et l'activateur externe 118 communiquent de façon sans-fil.

Comme illustré sur la vue éclatée de la figure 2, le dispositif d'urine 100 comprend une station 200 et une cartouche 202, montée amovible dans la station 200. La station 200 comprend notamment un boitier 204 qui est, selon une réalisation particulière, formé comme un assemblage de deux demi-coques : une coque avant 206 et d'une coque arrière 208. La coque avant et la coque arrière forment une jointure 210 du boitier, dans un plan normal à l'axe A. L'assemblage du dispositif d'analyse d'urine est facilité lorsque le boitier est constitué de la coque avant et de la coque arrière. Le boitier 204 renferme un ensemble de test (non visible sur la figure 2 mais visible sur la figure 6). L'ensemble de test est destiné à analyser l'urine étant reçue dans le dispositif d'analyse d'urine 100. La station 200 comporte en outre un logement annulaire 212, à l'intérieur du boitier 204, agencé autour d'un axe de rotation A. Le logement annulaire 212 est configuré pour recevoir au moins partiellement la cartouche 202 montée à rotation autour de l'axe de rotation A (une fois en position dans le logement annulaire 212). La cartouche 202 comprend une pluralité de supports de test intégrant un réactif, par exemple un réactif sec agencés le long d'un cercle ou d'un arc de cercle autour de l'axe de rotation A. Dans un mode de réalisation et pour la suite de la description, les supports de tests sont des bandelettes de test. En particulier, le logement annulaire 212 peut être partiellement délimité, avec jeu fonctionnel, par un couvercle interne 214, monté par exemple avec la coque arrière 208, pour protéger des composants de l'ensemble de test. Le couvercle interne 214 peut comprendre une portion radiale externe, pour protéger les composants radialement externes au logement annulaire 212 et une portion radiale interne, pour protéger les composants radialement internes au logement annulaire 212.

Le logement annulaire 212 s'étend typiquement sur 360° et forme une gorge configurée pour recevoir en partie la cartouche 202.

Dans la présente description, la locution « radialement interne au logement annulaire signifie plus proche de l'axe de rotation A que le logement annulaire n'est proche de l'axe de rotation A. De façon similaire, la locution « radialement externe au logement annulaire signifie « plus éloigné de l'axe de rotation A que le logement annulaire n'est éloigné de l'axe de rotation A ».

Le logement annulaire 212 est accessible par exemple en détachant la coque avant 206 de la coque arrière 208. Les coques avant 206 et arrière 208 peuvent être vissées l'une à l'autre à l'aide d'un filetage 216.

La station 200, en particulier le boitier 204, comprend en outre un orifice de collecte 218, positionnée par exemple sur la coque arrière 208 sur la figure 2. L'orifice de collecte 218 peut recevoir l'urine ruisselant par gravité sur la surface extérieure du boitier 204. Plus de détails sur cet orifice de collecte 218 seront donnés par la suite.

Le boitier 204 est agencé dans la cuvette 106 des toilettes de manière amovible. Le dispositif d'analyse 100 peut alors être retiré ou repositionné dans les toilettes. En outre, le dispositif d'analyse d'urine 100 ou le boitier 204 peut être retiré pour recharger une batterie ou pour changer la cartouche 202.

Dans l'exemple illustré à la figure 2, le boitier 204 est agencé sur la paroi interne 112 des toilettes. Le boitier 204 est positionné par un élément de fixation 300, dont un mode de réalisation est visible notamment sur la figure 3. L'élément de fixation 300 peut comporter des aimants 302 et/ou une surface adhésive/à ventouse qui peut coopérer. Cette configuration permet de facilement retirer ou repositionner le boitier dans les toilettes. Dans une autre configuration, le dispositif d'analyse d'urine 100 comprend un crochet solidaire à une extrémité du boitier 204 et configuré pour s'attacher à l'autre extrémité à un rebord de la cuvette 106 (sous le siège 108 par exemple).

La station 200, en particulier le boitier 204, comprend en outre un orifice de vidange 310, positionnée sur la coque arrière 208 sur la figure 3. L'orifice de collecte 218 peut recevoir l'urine ruisselant par gravité sur la surface extérieure du boitier 204 et l'orifice de vidange 310 permet de vidanger les différents fluides captés par le dispositif 100. Plus de détails sur cet orifice de vidange 310 seront donnés par la suite.

### Caractéristiques générales du boitier

Comme représenté sur les figures 2 à 4, le boitier 204 a une forme extérieure de galet circulaire. En d'autres termes, le boitier a une forme de sphéroïde aplatie. L'axe de rotation A est l'axe médian du boitier. Le boitier comporte une face avant 304 et une face arrière 306, sensiblement normales à l'axe A. La face avant 304 comprend typiquement la surface externe de la coque avant 206 et la face arrière 306 comprend notamment la surface externe de la coque arrière 208. Ainsi, une collecte d'urine peut se faire directement depuis les faces 304, 306 du boitier. Le boitier 204 sert de collecteur d'urine.

La face avant 304 est orientée vers l'intérieur de la cuvette 106. La face avant 304 est alors destinée à recevoir de l'urine lorsque l'utilisateur urine en position assise sur les toilettes. La face arrière 306 est orientée face à la paroi intérieure 112 de la cuvette 106. La face avant 304 et la face arrière 306 sont reliées par des bords courbés 308. Alors, la surface extérieure du boitier 204, constituée de la face avant 304, la face arrière 306 et les bords courbés 308, est définie par des lignes courbes, formant un objet généralement convexe. Le boitier est par exemple dépourvu d'arêtes. De l'urine peut ruisseler sur l'ensemble de la surface extérieure du boitier sans décrocher du boitier ou former de bulles d'air, pouvant compromettre une analyse de l'urine. La demande PCT/EP2021/055377 décrit en détail la forme du boitier 204 pour permettre une captation efficace de l'urine.

Dans un mode de réalisation, le boitier 204 a un diamètre, mesuré dans la direction normale à l'axe A, compris entre 50 mm et 150 mm, par exemple voisin de 100 mm. Le boitier 204 a également une épaisseur, mesuré selon la direction de l'axe A, comprise entre 15 mm et 50 mm, par exemple voisine de 30 mm. Ainsi, le boitier est suffisamment compact pour être entièrement reçu dans la cuvette des toilettes. Le dispositif d'analyse d'urine est discret. En outre, le boitier est suffisamment étendu pour systématiquement entrer en contact avec de l'urine étant reçu dans la cuvette. L'utilisateur peut alors uriner dans les toilettes sans se soucier du dispositif d'analyse d'urine, ou à défaut viser sommairement.

La surface extérieure du boitier est lisse. Ainsi, le jet d'urine entrant en contact avec le boitier s'accroche et s'étale sur les surfaces extérieures du boitier. Dans un mode de réalisation, le boitier est d'un matériau hydrophile. Par exemple, le boitier peut être en un parmi : une céramique, un polyamide (PA), un silicone ou un polymère hydrophile. La surface extérieure du boitier peut également être traitée par un traitement de surface hydrophile, par exemple acuWet^{®} de la société Aculon, un polymère hydrophile, ou Pebax^{®} de la société Arkema.

La coque avant 206 et la coque arrière 208 sont assemblées pour maintenir la surface extérieure du boitier définie par des lignes courbes. Alors, la jointure 210 entre la coque avant 206 et la coque arrière 208 permet un ruissellement d'urine entre la face avant et la face arrière. L'impact de la jointure sur l'écoulement d'urine sur le boitier est minimisé.

Alternativement au vissage, la coque avant 28 et la coque arrière 30 peuvent être assemblées par collage, clipsage, par aimantation, par baïonnette ou soudure ultrason. Bien entendu d'autres moyens de fixation peuvent être mis en oeuvre pour assembler la coque avant et la coque arrière. Dans le cas du vissage, une portion interne de la coque avant comporte un filetage. Le filetage de la coque avant est destiné à coopérer avec un taraudage de la coque arrière, ou inversement. Le boitier peut ainsi facilement être démonté pour accéder à l'ensemble de test à l'intérieur du boitier.

Un joint d'étanchéité peut être présent au niveau de la jointure 210 entre la coque avant et la coque arrière. Ainsi, le boîtier est étanche. L'intérieur du boitier 204 est imperméable à l'urine, à l'eau provenant du réservoir d'eau 104 ou de la cuvette 106, et tout autre type de contaminant. Seuls des orifices de collecte et de vidange relient l'extérieur et l'intérieur du boitier, comme décrit plus en détail plus loin.

Dans un mode de réalisation non illustré, la coque avant 206 ou la coque arrière 208 comprend un capot amovible permettant le remplacement de la cartouche 202. Plutôt que de démonter la coque avant 206 pour accéder au logement annulaire 212, il suffit alors d'enlever le capot amovible.

Le capot amovible peut être fixé sur la coque arrière 208 par clipsage, vissage ou par un mécanisme de baïonnette. Bien entendu, d'autres moyens de fixation peuvent être mis en oeuvre pour fixer le capot amovible sur la coque arrière 208. Alternativement, dans un autre exemple, le capot amovible pourrait être fixé sur la coque avant 206.

Le capot amovible est agencé de manière étanche. Par exemple, une jointure entre le capot amovible et la coque arrière 208 peut comporter un joint d'étanchéité. L'intérieur du boitier 204 reste ainsi imperméable à l'urine, à l'eau provenant du réservoir d'eau 104 ou de la cuvette 106, et tout autre type de contaminant.

Dans l'exemple illustré notamment à la figure 2 et ainsi qu'il l'a déjà été mentionné, le capot amovible est formé par la coque avant 206 du boitier 204. Le capot amovible peut alors être retiré par dévissage de la coque avant 28 par rapport à la coque arrière 30. Le boitier 204 comporte moins de jointures pouvant être salies et/ou infiltrées par l'eau des toilettes.

Le boitier 204 présente un orifice de collecte 218, déjà présenté en relation avec la figure 2. L'orifice de collecte 218 peut recevoir l'urine ruisselant par gravité sur la surface extérieure du boitier. Une collecte d'urine est réalisée directement depuis les faces 305, 306 du boitier.

L'orifice de collecte 218 est situé sur une extrémité inférieure 402 du boitier 204. L'extrémité inférieure 404 est orientée vers le fond de la cuvette 106 lorsque le boitier 204 est positionné dans la cuvette 106 des toilettes. Cette position correspond à une position normale d'utilisation. Cette position permet une collecte de l'urine ruisselant par gravité sur la majorité de la surface extérieure du boitier.

En l'espèce, une distance D séparant l'orifice de collecte 218 d'une bordure inférieure 404 du boitier est inférieure à 40 mm, par exemple inférieure à 20mm. Selon une réalisation particulière, l'orifice de collecte 218 est agencé à quelques millimètres au-dessus de la bordure inférieure du boîtier. En variante, l'orifice de collecte peut être sur la bordure inférieure 404.

L'orifice de collecte 218 est une ouverture circulaire, de diamètre par exemple compris entre 0,3 mm et 2 mm. Le diamètre de l'orifice de collecte peut être choisi afin de maximiser le volume d'urine collecté de la surface extérieure du boitier.

Le boitier 204 présente un orifice de vidange 310, déjà présenté en relation avec la figure 4. L'orifice de vidange 310 permet de purger le dispositif d'analyse d'urine 100 d'un excès d'urine.

L'orifice de vidange 310 est distinct de l'orifice de collecte 218. L'orifice de vidange 310 est également situé sur l'extrémité inférieure 404 du boitier 204, au voisinage de l'orifice de collecte 218. L'orifice de vidange 310 est également une ouverture circulaire. L'orifice de vidange 310 a un diamètre compris entre 0,3 mm et 2 mm. Dans la position normale d'utilisation, l'orifice de vidange 310 peut se trouver au-dessus de l'orifice de collecte 218 sans qu'il n'y ait de contamination croisée.

L'orifice de vidange 310 peut également être éloigné de l'orifice de collecte 32. La position de l'orifice de vidange 310 peut être choisie pour faciliter l'accès à l'orifice de vidange par l'ensemble de test 24.

En variante, l'orifice de vidange peut être le même que l'orifice de collecte. Un unique orifice permet de limiter le nombre d'ouvertures vers l'intérieure du boitier. Alors, le risque d'introduction de contaminants ou d'éléments susceptibles d'obstruer l'ensemble de test est réduit.

L'orifice de collecte 218 et/ l'orifice de vidange 310 peuvent être équipés d'un filtre en maille métallique. L'ouverture moyenne de maille du filtre est par exemple de 20 microns. Le filtre permet d'empêcher l'introduction de contaminants ou d'éléments susceptibles d'obstruer l'ensemble de test, et de filtrer l'urine reçue dans l'orifice de collecte.

Dans les exemples illustrés, l'orifice de collecte 218 et l'orifice de vidange 310 sont situés sur la face arrière 306 du boitier (sur la coque arrière 208). L'orifice de collecte 218 et l'orifice de vidange 310 font face à la paroi intérieure de la cuvette lorsque le dispositif d'analyse d'urine 100 est positionné dans les toilettes. Cette position permet de masquer l'orifice de collecte et l'orifice de vidange par la face avant du boitier. Aussi, cette position permet d'empêcher l'introduction de contaminants ou d'éléments susceptibles d'obstruer l'ensemble de test.

Le boitier 204 ne se limite pas aux seuls modes de réalisation décrits ci-avant en regard des figures, mais est, au contraire, susceptible de nombreuses variantes accessibles à l'homme de l'art.

Le boitier peut notamment prendre toute forme géométrique définie par des lignes courbes. Le boitier peut notamment avoir une forme de losange ou de goutte inversée. Alors, le boitier comporte une pointe sur la partie inférieure afin de guider l'urine vers l'orifice de collecte.

L'orifice de collecte et l'orifice de vidange peuvent être sur la face avant du boitier. Ainsi, l'urine ruisselant sur la face avant atteint l'orifice de collecte de manière plus directe.

L'orifice de collecte et l'orifice de vidange peuvent être situés sur un relief positif, notamment une saillie, ou négatif, notamment une gouttière ou un renfoncement. De manière générale, le relief peut être de toute géométrie permettant de canaliser l'urine ruisselant sur le boitier et de l'acheminer vers l'orifice de collecte sans décrocher du boitier ou former des bulles d'air.

Dans un exemple de réalisation, l'orifice de collecte 218 est agencé sur la face avant 304, alors que l'orifice de vidange 310 est situé sur la face arrière 306.

### Ensemble de test (injecteur et analyseur)

Une cartouche et un ensemble de test permettant d'exploiter des supports de test de la cartouche, comme bandelettes (par exemple des bandelettes colorimétriques ou des *lateral flow),* vont être décrit (plus de détails en fin de description). Les bandelettes sont ici également désignées « bandelettes de tests » ou plus simplement « bandelette ». L'ensemble de test comprend notamment un injecteur et un analyseur (par exemple un analyseur optique). La cartouche 202 est entrainée en rotation dans le boitier 204 par un actionneur d'entrainement, positionné dans le boitier 204, de sorte que les bandelettes de test peuvent défiler successivement devant l'injecteur et l'analyseur. L'ensemble de test comprend également un capteur de position, pour identifier une position des bandelettes dans le boitier. Le capteur de position peut permettre d'identifier localement la position de la bandelette (et pas nécessairement en absolu). Selon les modes de réalisation, et en particulier des modes de réalisation qui seront décrits par la suite, l'injecteur, l'analyseur et/ou le capteur de position peuvent être positionnés dans le boitier 204 au même endroit angulairement ou bien à des positions angulaires différentes, soit radialement internes ou externes au logement annulaire 212.

L'injecteur permet d'injecter un volume contrôlé d'urine sur une bandelette lorsque ladite bandelette est dans une zone d'injection ZI du logement annulaire 212. La zone d'injection ZI correspond à une fenêtre angulaire (autour de l'axe de rotation) du logement annulaire 212 à l'intérieur de laquelle l'injecteur est en mesure d'injecter l'urine sur la bandelette.

L'analyseur permet d'analyser une bandelette lorsque ladite bandelette est dans une zone d'analyse ZA du logement annulaire 212. La zone d'analyse correspond à une fenêtre ou plage angulaire (autour de l'axe de rotation) à l'intérieur de laquelle l'analyseur est en mesure de prendre des mesures sur la bandelette.

Le capteur de position permet d'obtenir une position de la cartouche ou de la bandelette lorsqu'un marqueur associé à celle-ci se trouve dans une zone de contrôle ZC. La zone de contrôle ZC correspond à une fenêtre ou plage angulaire (autour de l'axe de rotation) à l'intérieur de laquelle le capteur de position est en mesure d'obtenir une position de la cartouche ou de la bandelette. En particulier, la mesure de position obtenue par le capteur de position est utilisée pour le pilotage du moteur d'entrainement (boucle de rétroaction). Dans un mode de réalisation, le capteur de position peut permettre à la station de savoir qu'une bandelette est bien dans la zone de contrôle, mais pas forcément de savoir de quelle bandelette il s'agit. Dans un autre mode de réalisation, le capteur de position, couplé à un comptage du déplacement à partir d'un zéro (décrit plus tard), peut permettre de savoir quelle bandelette exactement se trouve dans la zone de contrôle.

Selon les modes de réalisation qui seront décrits par la suite, la zone d'injection ZI, la zone d'analyse ZA et/ou la zone de contrôle ZC peuvent être confondues, partiellement confondues ou bien distinctes.

En particulier, l'analyseur peut détecter un changement de couleur du support de test, qui peut être une bandelette (analyse colorimétrique). L'analyseur peut alors être un analyseur optique, avec une source de lumière et un capteur optique.

### Cartouche

La figure 5 illustre une vue éclatée d'un mode de réalisation de la cartouche 202. La cartouche 202 intègre des supports de tests destinées à recevoir de l'urine lorsque la cartouche 202 est montée dans la station 200 et, en particulier, dans le logement annulaire 212. Le support de test comprend un réactif qui réagit une fois en contact de l'urine. Comme illustrés sur les figures et comme, les supports de tests peuvent être des bandelettes de tests 501 (pour le reste de la description on parlera de bandelettes de tests) .La cartouche 202 comprend un support rotatif 500, configuré pour être entrainé en rotation par la station 200. En utilisation normale de la cartouche 202 et du dispositif d'analyse d'urine 100, les bandelettes 501 demeurent montées dans le support rotatif 500 et ne se déplacent pas par rapport à ce dernier. Les bandelettes sont donc attachées et solidaires du support rotatif 500. En particulier, les bandelettes 501 ne sont pas enroulées de façon déroulable sur le support rotatif 500 pour l'utilisation : elles ne sont donc pas déroulées pendant l'utilisation.

Dans un mode de réalisation, le support rotatif 500 est de forme cylindrique creuse s'étendant annulairement autour d'un axe qui est, lorsque la cartouche 202 est montée dans la station 200, l'axe médian A du boitier 204 (par commodité de langage, on utilisera un seul axe A pour décrire les différents éléments, appelé l'axe de rotation A). En pratique le support rotatif est généralement symétrique de révolution autour de l'axe de rotation A. Le support rotatif 500 permet de stocker un nombre important de bandelettes de tests 501 tout en étant suffisamment compact pour être agencé à l'intérieur du boitier 204.

La cartouche 202 et le support rotatif 500 tels qu'illustrés sur les figures s'étendent sur un tour complet et peuvent effectuer dans la station 200 un tour complet. Toutefois, il peut être envisagé, pour des raisons d'encombrement ou pour permettre de libérer de l'espace pour d'autres composants, une cartouche 202 qui s'étend sur une portion de tour (par exemple moins de 180° ou 90°) et qui n'effectue en rotation qu'une portion de tour (par exemple moins de 270°). Dans ce cas, le nombre de bandelettes est typiquement moins élevé que pour le dispositif d'analyse d'urine illustré sur les figures.

Les bandelettes sont agencées selon un cercle ou une portion de cercle, par exemple à une extrémité radiale du support rotatif 500 pour maximiser leur nombre (plus le rayon est grand, plus le périmètre pour y installer des bandelettes est grand). Le positionnement en cercle assure que les bandelettes 501 sont toutes à la même distance de l'axe de rotation A et, de ce fait, de l'injecteur ou de l'analyseur (en particulier un capteur optique de l'analyseur, qui sera décrit plus tard). Cela assure aussi que le protocole de mesure pour chaque bandelette est identique. Comme illustré sur les figures, les bandelettes 501 peuvent être agencées généralement selon une disposition en cercle ou en arc de cercle. Ainsi les bandelettes 501 peuvent donc être à équidistance de l'axe de rotation A. Plus spécifiquement, chacune des bandelettes 501 peut être une petite bande peu épaisse et peu large qui s'étend dans son sens longitudinal parallèlement à l'axe A. Ainsi les bandelettes 501 sont disposées parallèlement entre elles.

Par sa forme et sa fonction, la cartouche 202 s'apparente à un barillet. Dans un exemple de réalisation, la cartouche occupe substantiellement le volume annulaire procuré par le logement annulaire 212. On prévoit un petit jeu fonctionnel pour permettre à la cartouche de tourner sans frotter contre les parois du logement annulaire 212.

En l'espèce, un diamètre extérieur du support rotatif 500 peut être compris entre 30 mm et 130 mm, de préférence environ 60 mm. Une hauteur du support rotatif, mesurée selon la direction de l'axe A peut être compris entre 12 mm et 40 mm, de préférence environ 14 mm. Un rapport entre le diamètre du support rotatif et le diamètre du boitier 204 peut être supérieur ou égal à 0,3, de préférence supérieur ou égal à 0,5. On obtient ainsi une solution de très bonne compacité au regard du nombre important de bandelettes de tests disponibles.

Le support rotatif 500 comprend une portion annulaire 502 et une portion cylindrique 504, s'étend depuis une extrémité radiale externe de la portion annulaire 502. La portion cylindrique 504 s'étend typiquement d'un seul côté de la portion annulaire 502 et est configurée pour s'insérer dans le logement annulaire 212 du boitier 204. Les bandelettes 501 sont positionnées le long de la portion cylindrique 504 (orientées parallèlement à l'axe de rotation A), afin de pouvoir défiler sélectivement et/ou successivement devant l'injecteur et l'analyseur. La portion annulaire 502, quant à elle, reste en dehors du logement annulaire 212 et permet notamment de rigidifier la portion cylindrique 504 et/ou de permettre l'entrainement en rotation de la cartouche 202.

A cet effet, la portion annulaire 502 peut comprendre en outre un coupleur mécanique 506 configuré pour s'engager avec un coupleur mécanique de la station 200, par exemple un manchon femelle de fixation configuré pour s'engager avec un arbre entrainé en rotation par le moteur ou bien un arbre mâle de fixation configuré pour s'engager un manchon femelle entrainé en rotation par le moteur. Dans l'exemple illustré aux figures, il est prévu un pignon axial d'entrainement 602 (visible en figure 6) en sortie de réducteur, et la portion femelle est formée par le moyeu 506 du support rotatif 500.

Dans un mode de réalisation, le coupleur mécanique 506 est disposé sur l'axe de rotation A du support rotatif 500. Toutefois, dans l'exemple où la cartouche 202 est entrainée en rotation via un coupleur sur la portion cylindrique, la portion annulaire 502 peut être dépourvue du manchon femelle 602. La portion annulaire 502 pourrait être montée par tout type de liaison pivotante par rapport au boitier 204. La portion annulaire 502, à l'exception du coupleur mécanique 506, lorsqu'il est traversant, au niveau de l'axe A, peut s'apparenter à un disque. Le manchon femelle et le moyeu peuvent être inversés.

Dans un mode de réalisation, la cartouche 202 comprend un séparateur 508 comportant des logements 510 permettant de recevoir les bandelettes 501. Les logements 510 et les bandelettes 501 ont des dimensions similaires qui seront données en fin de description.

Le séparateur 508 est par exemple une pièce flexible, notamment en élastomère, en forme de bande ou ruban destinée à être enroulée dans le support rotatif 500. Le séparateur 508 s'étend le long d'une direction longitudinale et peut être enroulés contre une paroi interne de la portion annulaire 504 du support rotatif 500. Le séparateur 508 comprend une première face comprenant une pluralité de logements 510 recevant chacun un ou plusieurs bandelettes de test 501. La première face peut être recouverte par un opercule pour protéger les bandelettes hors utilisation. Le séparateur 508 comprend une deuxième face comprenant, en regard de chaque logement 510, au moins un orifice traversant 512 (par exemple deux, comme illustré sur la vue en transparence de la portion annulaire 504). Les logements 510 sont ainsi scellés. Grâce au séparateur 508 flexible, l'insertion des bandelettes de test 501 dans les logements 510 peut se faire sur une surface plane, ce qui simplifie le montage. Une fois le séparateur 508 installé, les logements 510 s'étendent parallèlement à la direction de rotation A.

La portion cylindrique 504 est transparente, ou bien comprend des zones transparentes, notamment en regard des logements 510. La portion cylindrique 504 est au contact du séparateur 508, en particulier la deuxième face du séparateur 508. Lors d'une analyse par colorimétrie, la lumière peut traverser la portion cylindrique 504, en passant par l'orifice traversant 512 pour analyser les bandelettes de test.

La portion cylindrique 504 peut notamment être en polycarbonate. En effet, le polycarbonate présente des bonnes propriétés de transmission de la lumière, tout en restant relativement peu coûteux et compatible avec un procédé de moulage par injection.

La portion annulaire 502 du support rotatif 500 forme un socle pour porter le séparateur 508. La portion cylindrique 504 reçoit le séparateur 508. Le séparateur 508 est bloqué en translation selon la direction A par un contact avec la portion annulaire 502. De l'autre côté, un rebord annulaire s'étendant depuis une extrémité de la portion cylindrique 504 et radialement vers l'intérieur permet de bloquer la translation selon l'autre sens de la direction A.

Le support rotatif 500, et plus particulièrement la portion cylindrique 504, comprend en outre une ouverture de purge 514, traversante, pour permettre à l'injecteur de traverser la cartouche 202 et le logement annulaire 212 et de rejoindre un circuit d'évacuation de la station. Le séparateur 508 ne recouvre pas l'ouverture de purge 514. Ce circuit sera décrit en détail plus tard.

La cartouche 202 comprend en outre un identificateur 516, représenté sur la figure 5 par une puce RFID. L'identificateur 516 permet à la station 200 de savoir quelle cartouche 202 a été insérée. L'identification 516 est typiquement un tag passif RFID.

Selon un autre exemple non illustré, le support rotatif 500 pourrait être une rondelle, d'axe confondu avec l'axe médian A du boitier. La rondelle s'étend alors radialement, selon un plan sensiblement normal à l'axe A médian du boitier. Les bandelettes de test peuvent alors être stockées sur une face de la rondelle, normale à l'axe A. Cette configuration permet notamment d'adapter le support rotatif à différentes formes de boitier. Alors, le support rotatif peut être implémenté dans divers analyseurs d'urine.

Chaque logement 510 peut recevoir une unique bandelette de test. Toutes les bandelettes de test 501 des logements 510 peuvent être d'un même type. Par même type, on entend qu'elles sont sensibles aux mêmes composés contenus dans l'urine. La cartouche 202 est alors adapté pour une analyse spécifique.

En variante, la bandelette de test 501 reçue dans le logement 510 peut être d'un type différent de la bandelette de test reçue dans le logement voisin. Ainsi, plusieurs types d'analyses, requérant différents types de bandelettes de test, peuvent être réalisées à partir de la même cartouche 202.

En variante, chaque logement 510 peut contenir une pluralité de bandelettes de test 501 de types différents. Ainsi, plusieurs types d'analyses peuvent être réalisées à partir d'un même logement.

Comme indiqué précédemment, chaque logement 510 est recouvert et fermé par un opercule (visible en figure 12 à la référence 511). L'opercule permet d'isoler hermétiquement les bandelettes de test 501 reçues dans un logement 510 de l'environnement extérieur et de logements voisins. Le logement 510 devient accessible pour l'injection de liquide typiquement par percement de l'opercule (par exemple par l'injecteur 604. Alors, avant une analyse, les réactifs des bandelettes de test sont protégés d'une éventuelle contamination. En outre, après une analyse, l'opercule peut contenir de l'urine introduite dans le logement 510. L'opercule peut prendre la forme d'un film continu. Le film est collé sur la paroi extérieure du support rotatif pour recouvrir les logements 510. Cette configuration facilite la mise en place de l'opercule 511 sur le support rotatif 500. Alternativement, chaque logement 510 peut être recouvert par un opercule distinct. Cette configuration permet de limiter le risque de contamination de bandelettes de tests reçues dans deux logements voisins. Alternativement encore, les bandelettes de test peuvent être encapsulées individuellement. Cette configuration apparait particulièrement intéressante lorsque les bandelettes de test sont reliées pour former un ruban. En effet, le ruban peut être assemblé dans le support rotatif sans nécessiter l'ajout d'un opercule supplémentaire. L'assemblage du dispositif d'analyse d'urine 100 est facilité. L'opercule est ici en un matériau inerte. Par exemple, l'opercule peut être en silicone ou en acrylique. De préférence, l'opercule est de qualité médicale, pour éviter la contamination des bandelettes de tests avec des produits indésirables contenus dans l'opercule. Alors, les réactifs des bandelettes de test sont conservés intacts avant une analyse. En outre, l'opercule est transparent, un taux de transparence étant de préférence supérieur à 99%. Alors, une analyse par colorimétrie peut être réalisée sur une bandelette de test au travers l'opercule. Actionneur d'entrainement

La cartouche 202 est, lorsque mise en place dans la station 200, couplée mécaniquement à un actionneur d'entrainement 600 (figures 6, 8 et 10), via son coupleur mécanique 506 et un coupleur mécanique complémentaire 602 de la station 200, pour être entrainé en rotation autour de l'axe A. La cartouche 202 peut alors être sélectivement positionnée pour aligner une bandelette de test face à l'injecteur, référencé 604 ou l'analyseur, référencé 606. L'usage de la cartouche 202 permet d'avoir un équipage mobile simple avec un seul axe de rotation. En outre, cette configuration réduit les contraintes liées au positionnement et l'agencement de l'injecteur 604 et de l'analyseur 606 dans le dispositif d'analyse 100.

L'actionneur d'entrainement 600 peut être déporté par rapport à l'axe A et un train d'engrenages 608 (pouvant aussi faire office de réducteur) permet d'entrainer le coupleur mécanique complémentaire 602. Le coupleur mécanique 602 complémentaire de la station 200 se trouve sur l'axe de rotation A.

L'actionneur d'entrainement 600 peut entrainer la cartouche 202 selon la direction horaire ou antihoraire. La cartouche peut alors rapidement atteindre la position désirée, suivant la trajectoire la plus courte. Ainsi, on réduit encore les contraintes liées au positionnement et l'agencement de l'injecteur 604 et de l'analyseur 100.

L'actionneur d'entrainement 600 est par exemple un moteur d'entrainement, comme un moteur pas-à-pas ou un moteur à courant continu. Alternativement, l'actionneur d'entrainement 600 peut impliquer un système hydraulique ou autre.

### Circuit fluidique

L'orifice de collecte 218 est fluidiquement connecté à un collecteur 610 puis un tuyau de collecte 612 puis une pompe 614, puis un tuyau d'acheminement 616 puis l'injecteur 604. L'injecteur 604 est mobile dans le boitier 204, de sorte que l'injecteur peut se déplacer pour injecter de l'urine sélectivement sur une bandelette ou dans un tuyau de vidange 618 relié à l'orifice de vidange 310, en fonction de la position de la cartouche 202 dans la station 200. L'urine collectée par l'orifice de collecte 218 est mise en mouvement dans le circuit fluidique grâce à la pompe 614. Le tuyau d'acheminent 616 peut comprendre une portion souple (non représenté sur la figure 6 par exemple), notamment à son extrémité qui le connecte à l'injecteur, pour accommoder le déplacement de l'injecteur. L'injecteur 604 comprend typiquement trois positions : une position de retrait PR, une position d'injection PI et une position de purge PP. En position de retrait PR, l'injecteur 604 ne gêne pas la rotation de la cartouche 202 dans le logement annulaire 212 ; en position d'injection PI, l'injecteur 604 permet l'injection d'urine sur une bandelette positionnée dans la zone d'injection ZI ; en position de purge PP, l'injecteur 604 est relié au tuyau de vidange 618. Typiquement, en position de retrait PR, l'injecteur 604 est entièrement radialement interne au logement annulaire 212, entièrement en retrait ; en position d'injection PI, l'injecteur 604 est partiellement interne au logement annulaire 212 et partiellement dans le logement annulaire 212 ; en position de purge, l'injecteur 604 est partiellement interne au logement annulaire 212, partiellement dans le logement annulaire 212 et partiellement externe au logement annulaire 212. Pour que l'injecteur puisse être en position de purge PP, la cartouche 202 doit être elle aussi être en position de purge, c'est-à-dire que l'orifice de purge 514 du support rotatif 500 doit être aligné avec l'injecteur 604 (radialement aligné avec une extrémité distale d'injection de l'injecteur).

La pompe 614 peut aspirer de l'urine. La pompe aspire par exemple entre 5 microlitres et 1 mL, de préférence environ 20 microlitres. De plus, la pompe permet d'acheminer un volume suffisant d'urine vers l'injecteur 604 pour pouvoir réaliser une analyse concluante. Un débit d'aspiration de la pompe 614 est choisi en fonction du diamètre de l'orifice de collecte. Avantageusement, la pompe peut aspirer de l'urine depuis l'orifice de collecte vers l'injecteur sans former de bulles d'air.

Selon une autre phase, après miction et hors séquence de chasse d'eau, la pompe 614 peut également aspirer de l'air depuis l'orifice de collecte 218 pour l'éjecter via l'orifice de vidange 310. Alors, la pompe permet d'expulser de l'urine ou de l'eau contenue dans le dispositif d'analyse d'urine. De l'urine collectée pour une analyse est alors protégée d'une éventuelle contamination par l'eau des toilettes ou d'une collecte précédente. Selon une autre phase ou une variante de réalisation, il peut être prévu que la pompe 614 puisse aspirer de l'eau lors de l'activation de la chasse d'eau des toilettes pour évacuer l'urine contenue dans dispositif d'analyse d'urine.

La pompe 614 peut être de différents types possibles. La pompe 614 peut être une pompe péristaltique miniaturisée. La pompe 614 peut être un système de pompe pneumatique miniaturisée comme détaillé ci-après. Dans le cas où la pompe 614 est une pompe pneumatique miniaturisée, ce système pneumatique est configuré pour créer une dépression afin d'aspirer l'urine depuis l'orifice de collecte 218, puis une pression positive pour pousser l'urine vers l'injecteur 604 et le canal de purge. La pompe du système pneumatique peut être ici une pompe de type rotative, le sens de rotation fournissant respectivement et sélectivement une dépression ou une surpression. La pompe du système pneumatique peut être aussi une pompe de type piézoélectrique.

Lorsque de l'urine est aspirée, il peut être prévu que l'injecteur soit en position de purge pour évacuer de l'air jusqu'à ce que de l'urine vienne en précharge.

La solution présentée permet de contrôler de manière précise le volume acheminé dans le dispositif d'analyse d'urine.

### Injecteur courbé

En relation avec les figures 6 à 8, l'injecteur 604 va être décrit plus en détail. L'injecteur 604 comprend une extrémité distale, dite d'injection, 700 et une extrémité proximale 702, dite de connexion, qui est typiquement configuré pour être connectée au tuyau d'acheminement 616 (en particulier la portion flexible du tuyau d'acheminement, qui n'est pas représentée sur la figure 6, de sorte que l'extrémité proximale 702 débouche à l'air libre). L'injecteur 604 peut comprend une aiguille 704 et l'extrémité distale 700 peut alors être l'extrémité de l'aiguille 704.

Selon un mode de réalisation alternatif, en référence à la figure 18, l'injecteur 604 comprend une connexion intermédiaire 1802, disposée entre l'extrémité proximale 702 et l'extrémité distale 700. Dans l'exemple de la figure 18, la connexion intermédiaire 1802 est arrangée au voisinage de l'aiguille 704, en particulier dans le prolongement de l'aiguille 704 selon l'axe T2. La connexion intermédiaire 1802 est définie par un logement dans l'injecteur 604, le logement étant relié fluidiquement à l'aiguille 704. Dans ce mode de réalisation, le tuyau d'acheminement 616 est connecté à la connexion intermédiaire 1802, notamment via la portion flexible du tuyau d'acheminement, qui n'est pas représentée sur la figure 18. La portion flexible est propre à s'insérer dans le logement de la connexion intermédiaire 1802 et fixé par exemple par clip ou par vissage. L'injecteur 604 ne comprend alors pas de canal de circulation de l'urine depuis l'extrémité proximale 702, l'extrémité proximale 702 ne présentant pas d'ouverture.

Afin de pouvoir injecter de l'urine sur les bandelettes 501, l'injecteur 604 est mobile en translation selon une direction de translation DT par rapport au boitier 204. Un actionneur de déplacement 620 est prévu dans le boitier 204 pour faire déplacer l'injecteur. L'injecteur 604 est positionné, dans le boitier 204, radialement à l'intérieur du logement annulaire 212. De la même façon, l'actionneur de déplacement 620 est lui aussi positionné radialement à l'intérieur du logement annulaire 212. Néanmoins, du fait de l'agencement des logements 510, la position angulaire acceptable pour l'injection (appelée fenêtre d'injection) est assez faible. Dans le cas de l'injecteur décrit dans le document PCT/EP2021055302 (à la figure 20 en particulier), la fenêtre est contrainte par l'inclinaison entre une direction radiale (selon un rayon du cercle défini par les logements) et une profondeur du logement. Par conséquent, le nombre de pas de moteur d'entrainement à activer pour que le logement soit correctement positionné lors de l'injection est faible.

L'injecteur 604 illustré en figures 6 à 8, et 18, ne présente pas ces difficultés. Selon un aspect, l'injecteur 604 présente une forme courbée, afin que l'axe de translation au niveau duquel l'injecteur 604 est entrainé en translation soit décalé par rapport à l'axe de translation de l'extrémité distale 700. On entend dans le mode de réalisation des figures 6 à 8 qu'une portion de l'injecteur qui canalise le fluide est courbée (et que le trajet du fluide suit sensiblement cette courbe). On entend dans le mode de réalisation de la figure 18 qu'une portion de l'injecteur entre l'endroit où il est entrainé en translation et l'extrémité distale est courbée. En particulier, si on définit un premier axe T1, qui est l'axe au niveau duquel l'injecteur 604 reçoit le mouvement de translation et un deuxième axe T2, qui est celui du déplacement de l'extrémité distale 700, ces deux axes T1, T2 ne sont pas confondus lorsque vus du dessus ou du dessous (le long de l'axe de rotation A) or lorsque projetés dans un plan orthogonal à l'axe de rotation A. Cela permet de déporter l'actionneur de déplacement 620 dans une région moins encombrée du boitier 204. Le deuxième axe T2 est parallèle à la direction de translation DT.

Selon un aspect illustré par les figures 6 à 8, l'injecteur 604 peut aussi être défini par le décalage (appelé déport) dans le plan orthogonal à l'axe A entre l'extrémité distale 700 et l'extrémité proximale 702 de l'injecteur. Ce décalage est obtenu notamment par une forme courbée de l'injecteur 604.

Selon un aspect, l'injecteur 604 peut aussi être défini par le fait qu'il se trouve radialement à l'intérieur du logement annulaire 212 et que l'extrémité distale 700 se déplace en translation selon une direction radiale ou sensiblement selon une direction radiale (c'est-à-dire radiale à quelques degrés près, par exemple 10°, préférablement 5° et même 2° de part et d'autre de ladite direction radiale, voire 1°). Ce déplacement radial est permis par la forme courbée (en projection dans un plan orthogonal à l'axe de rotation A) de l'injecteur 604.

La forme courbée de l'injecteur 604 permet de contourner le coupleur mécanique 602 se trouvant au niveau de l'axe de rotation A tout en ayant le deuxième axe T2 qui soit confondu avec un rayon du cercle définir par les logements. En d'autres termes, l'extrémité distale 700 se déplace le long d'une direction radiale, et donc orthogonale à l'opercule 511, à la bandelette 501 et/ou au fond du logement 510. Par conséquent, la premier axe T1 ne traverse pas le coupleur mécanique 602 mais le deuxième axe T2 traverse le coupleur mécanique 602. Ainsi, l'injecteur 604 n'interfère pas avec un cylindre de rayon R4 centré sur l'axe A. Cela laisse libre un espace axial où vient se loger le moyeu de du support 500 de la cartouche.

Le premier axe T1 est décalé de l'axe T2 (dans un plan orthogonal à l'axe de rotation) d'une distance de déport égale à moins la distance du rayon R4 (R4 plus l'épaisseur d'un couvercle, plus un jeu fonctionnel au moins). Le rayon R4 peut être au moins égal à 3 mm. R4 peut être au moins égal à 5mm. La distance de déport est typiquement comprise entre 5 et 15 mm, voire entre 5 et 10mm.

Le deuxième axe T2 est avantageusement radial ou sensiblement radial (à plus ou moins 2°, voire 5°, voire 10° de part et d'autre une direction radiale).

Dans le mode de réalisation des figures 6 à 8, la courbure de l'injecteur 604 peut comprendre deux coudes successifs inversés 706, 708 (voir figure 7 notamment). En particulier, entre l'extrémité distale 700 et l'extrémité proximale 702, l'injecteur 604 comprend une portion intermédiaire 710. Les deux coudes 706, 708 peuvent être formés dans la portion intermédiaire 710. Les deux coudes 706, 708 sont arrondis, afin de perturber le moins possible le déplacement du fluide. Le coude proximal 708 (le plus proche de l'extrémité proximale 702) peut avoir une forme complémentaire au coupleur mécanique 604 de la station 200 pour permettre à l'extrémité distale 700 d'avoir une amplitude de translation maximale. A cette fin, le coude distal 706 (le plus proche de l'extrémité distale 700) peut avoir un rayon de courbure plus faible, pour que la portion intermédiaire soit majoritaire constituée par le coude proximal 708. Le coude distale 708 a donc pour fonction essentiel de permettre d'insérer l'injecteur 604 dans le boitier 204, en contournant le coupleur mécanique 602 et le coude proximal 706 a pour fonction de réaligner l'injecteur 604 (en particulier pour réaligner l'aiguille 704) dans une direction radiale ou sensiblement radiale.

Dans une variante, il s'agit de l'aiguille elle-même qui est courbe.

Dans un mode de réalisation, l'injecteur 604 est rigide. Par rigide, il est entendu qu'il ne se déforme pas lors de l'utilisation normale du dispositif. En particulier, la portion intermédiaire 710 est rigide.

En position de retrait PR, l'injecteur 604 peut être à moins de 2mm du coupleur mécanique 604 (distance la plus proche).

En position de purge PP, l'extrémité distale 700 de l'injecteur 604 se trouve radialement externe au logement annulaire 212. L'ouverture de purge 514 peut aussi servir de marqueur zéro pour l'obtention de la position la cartouche 202.

Dans un mode de réalisation, la courbure de l'injecteur 604 se fait uniquement dans un plan orthogonal à l'axe de rotation A. Dans un autre mode de réalisation, l'injecteur peut avoir une courbure qui crée un décalage le long l'axe de rotation A, de sorte que l'extrémité distale 700 soit plus haute ou plus basse (le long de l'axe de rotation A) que l'extrémité proximale 702. Cela permet de gérer des différences de niveau dans le dispositif 100.

Pour déplacer l'injecteur 604 en translation, plusieurs options sont possibles. L'actionneur de déplacement 620, monté dans le boitier 204 est prévu pour générer le mouvement. La figure 8 illustre un mode de réalisation à l'aide d'une liaison vis-écrou. L'actionneur de déplacement 620 peut être un moteur de déplacement 620, par exemple un moteur électrique, qui entraine en rotation une tige 622. La tige 622 coopère mécaniquement avec un écrou 800 solidaire de l'injecteur 604, de sorte que le mouvement de rotation de la tige autour du premier axe T1 se transforme en translation de l'écrou 800 au niveau du premier axe T1. La tige 622 et l'écrou 800 comprennent chacun un filetage qui coopère l'un avec l'autre. Du fait de la courbure de l'injecteur 604, l'encombrement de l'actionneur de déplacement 620, de la tige 622 et des autres composants permettant de réaliser un injecteur fonctionnel sont déportés dans une zone libre du boitier 204, à côté du coupleur mécanique 604 de la station 200, tandis que l'extrémité proximale 700 de l'injecteur 604 peut se déplacer le long du deuxième axe T2, qui est une direction radiale ou sensiblement radiale. L'écrou 800 est dit écrou prisonnier, en ce sens où il ne peut pas tourner sur lui-même.

La liaison vis-écrou permet une bonne conversion des mouvements, sans saut de pas, décalage entre la consigne et le résultat, ou même sans perte de liaison en cas de chute de la station 200. De plus, au vu des masses des éléments, les frottements et la nécessité de lubrification sont faibles. Par exemple, une lubrification lors de l'assemblage suffit.

L'écrou 800 peut être réalisé sous la forme d'un insert fileté ou par taraudage d'un orifice.

Alternativement, l'actionneur de déplacement 620 peut être un moteur linéaire, voire un actionneur linéaire (vérin, etc.), qui produit un mouvement en translation directement.

Pour améliorer la précision, l'injecteur 604 peut être solidaire d'un guide 802 (ou coulisse) en translation au niveau du premier axe T1. Le guide 802 peut comprendre un orifice circulaire qui coopère avec une tige fixe 804 solidaire du boitier 204. Le guide 802, quand il est sous la forme d'un orifice circulaire, ne peut pas se défaire de la tige fixe 804, ce qui assure une bonne robustesse (par exemple en cas de chute de la station 200).

L'injecteur 604 peut être monté sur un chariot 900, illustré seul en figure 9 et visible dans le boitier en figure 10. Le chariot 900 présente une forme courbée qui peut accueillir l'injecteur 604. La reprise d'effort de l'actionneur de déplacement 620 peut se faire sur le chariot 900 plutôt que sur l'injecteur 604 directement, pour préserver ce dernier de toute contrainte mécanique. En effet, le déport dû à la courbure de l'injecteur 604 et la force de réaction de l'opercule 511 lors de son percement par l'injecteur 604 peut créer un couple parasite. Le chariot 900 permet davantage de solidité du système. Par exemple, au niveau d'une extrémité proximale 902 du chariot 900 se trouve l'écrou 800 qui se couple avec la tige 622. Au niveau d'une extrémité distale 904 se trouve le guide 802, sous la forme d'un orifice circulaire. Le décalage entre les deux orifices de l'écrou 800 et du guide 802 peut correspondre au décalage entre le premier axe de translation T1 et le deuxième axe de translation T2 (dans un plan orthogonal à l'axe de rotation A). Le chariot 900 peut comprendre des clips de maintien 906 pour tenir l'injecteur 604 en position.

L'actionneur de déplacement 620 permet de déplacer l'injecteur 604 dans les trois positions mentionnées précédemment : la position de retrait PR, la position d'injection PI, la position de purge PP. Une mise en action de l'actionneur de déplacement 620 permet d'effectuer ces déplacements entre les trois positions PPI, PI, PP.

D'autres types de moteurs et/ou liaisons mécaniques permettent de déplacer l'injecteur 604 en translation.

L'extrémité distale 700 peut aussi être configurée pour percer l'opercule 511 de la cartouche 202. Un biseau peut être prévu pour faciliter l'insertion.

L'aiguille 704 peut avoir un diamètre d'environ 0,5mm. L'extrémité proximale 702 et la portion intermédiaire 710 peuvent être monobloc (par exemple réalisé par impression 3D, et l'aiguille 704 est inséré dans la portion intermédiaire qui comprend un orifice prévu à cet effet. L'aiguille 704 peut être en métal.

La zone d'injection ZI, qui correspond à la fenêtre ou la plage angulaire (autour de l'axe de rotation) de positionnement d'une bandelette 501 pour une bonne injection d'urine par l'injecteur 604, comprend typiquement quelques degrés maximum (moins de 5°, voire moins de 2°). Dans le cas d'un actionneur d'entrainement pas à pas (sous la forme d'un moteur d'entrainement 600 pas à pas par exemple), cette fenêtre d'injection correspond à quelques pas (le nombre dépendant du moteur d'entrainement 600 lui-même et du train d'engrenages 608).

L'injecteur 604 peut injecter un volume contrôlé d'urine sur une bandelette de test, par exemple entre 2,5 microlitres et 3,5 microlitres. L'injecteur injecte un volume suffisant d'urine sur une bandelette de test pour réaliser une analyse concluante sans risquer un débordement d'urine du logement.

L'injecteur 604 peut injecter un volume contrôlé d'urine sur la bandelette de test 56 en deux temps. Par exemple, l'injecteur 604 peut injecter deux fois entre 2,5 microlitres et 3,5 microlitres. Cette solution permet de prendre en compte un temps de réaction et de migration de l'urine sur la bandelette de test 501.

### Capteur de position et injecteur

Un capteur de position 624 peut être prévu dans le boitier 204 pour obtenir la position de la cartouche 202 et/ou des bandelettes 501 par rapport au boitier 204 et plus précisément par rapport à l'injecteur 604 et/ou l'analyseur 1200. Cela permet d'améliorer la précision puisque l'actionneur de déplacement 620 peut être piloté par des consignes de position utilisant les données du capteur de position 624 (boucle de rétroaction).

Afin que l'injecteur 604 puisse injecter l'urine correctement sur la bandelette 501 choisie, il est important de connaitre la position des bandelettes 501 dans le logement annulaire 212. En particulier, du fait de la structure de la cartouche 202, des imprécisions d'assemblage sont possibles, de sorte que les bandelettes 501 ne soient pas régulièrement positionnées (décalages faibles, mais au vu des distances impliquées, qui peuvent générer des mauvaises analyses).

Dans un mode de réalisation, la station d'analyse 200 comprend donc un capteur de position 624 (visible en figures 6 ou 11). Pour des raisons d'encombrement, le capteur de position 624 peut être positionné au moins partiellement, dans le boitier 204, radialement à l'extérieur du logement annulaire 212. Le capteur de position 624 permet d'identifier la position d'un marqueur de la cartouche 202 lorsque ledit marqueur se trouve dans la zone de contrôle ZC. Il y a typiquement au moins autant de marqueur que de bandelette 501, de sorte que chaque bandelette 501 puisse être repérée par un marqueur. Comme indiqué précédemment, l'injecteur 604 est configuré pour injecter de l'urine dans une zone d'injection ZI du logement annulaire 212 et le capteur de position 624 est configuré pour obtenir la position d'un marqueur associé à la bandelette 501 dans une zone de contrôle ZC du logement annulaire 212.

Dans un mode de réalisation, la zone d'injection ZI et la zone de contrôle ZC sont confondues ou à proximité. En d'autres termes, le capteur de position 624 permet de mesurer la position d'un marqueur associée à une bandelette 501 lorsque ledit marqueur est positionné dans la zone d'injection ZI ou dans une zone de proximité de la zone d'injection ZI (la mesure se fait sur ce marqueur, et non pas par déduction à partir d'une mesure sur un marqueur hors ou à distance de la zone d'injection ZI). Cela signifie que le capteur de position 624 permet de connaitre précisément la position d'une bandelette d'intérêt 501 dans la zone d'injection ZI et donc d'injecter l'urine correctement sur la bandelette d'intérêt 501. Les imprécisions de position des bandelettes 501, dues au positionnement des bandelettes dans le séparateur 508 et au séparateur 508 lui-même dans la cartouche 202 (qui est souple), sont généralement faibles d'un logement à l'autre. Par conséquent, en mesurant la position d'un marqueur dans une zone de proximité de la zone d'injection ZI et donc à proximité de la bandelette 501 d'intérêt, on obtient une donnée assez précise quant à la position de la bandelette 501 d'intérêt dans cette zone d'injection ZI. Par zone de proximité, il est signifié à moins de 30° de part et d'autre de la zone d'injection, voire moins de 20°, voire moins de 10°, voire moins de 5°. Plus le capteur de position 624 mesure un marqueur proche de la bandelette 501, plus la précision est bonne pour cette bandelette 501. A l'inverse, plus le capteur de position 624 mesure la position d'un marqueur éloigné de la zone d'injection ZI, plus les imprécisions de positionnement peuvent se cumuler, de sorte que la bandelette d'intérêt peut en réalité ne pas être correctement située dans la zone d'injection ZI.

Dans un mode de réalisation, le marqueur est la bandelette 501 directement, ce qui maximise la précision. Une description détaillée sera donnée plus loin. Alternativement, le marqueur est un élément repérable situé sur la portion cylindrique 504 de la cartouche 202 ou bien sur le séparateur 508.

Par exemple, comme illustré sur la figure 11, le capteur de position 624 est un capteur mécanique, par exemple sous la forme d'un suiveur 1100, monté dans le boitier 204, qui coopère avec une came lobée 1102 de la cartouche 202 (de la portion cylindrique 504 par exemple). Le marqueur est alors le haut 1104 ou le bas 1106 de la came lobée qui est le plus proche d'une bandelette. Ainsi, en mesurant la position du marqueur, qui est à proximité de la bandelette, la position de la bandelette peut être connue et cette dernière peut être correctement positionnée dans la zone d'injection ZI.

Par exemple, le capteur de position 624 peut comprendre un capteur électromagnétique, comme un capteur à effet Hall et le marqueur peut être un élément magnétique sur la cartouche. Un élément magnétique peut être monté sur le support rotatif en regard ou juste à côté de chaque logement 510.

Dans un mode de réalisation, le capteur de position 624 est radialement aligné (une fois projeté dans un plan orthogonal à l'axe de rotation) avec l'extrémité distale de l'injecteur (même emplacement angulaire autour de l'axe de rotation A donc), mais décalés le long de l'axe de rotation. De la sorte, la mesure obtenue est pertinente pour l'injection.

Dans un mode de réalisation, le capteur de position 624 est un capteur optique qui repère le marqueur par analyse d'un signal lumineux reçu. Plus spécifiquement, le capteur de position 624 peut être l'analyseur 606 lui-même. Cela permet de gagner en compacité et en précision, puisque le marqueur est très proche de la bandelette (ou est même la bandelette).

### Analyse et injection

La figure 12 illustre en détail un mode de réalisation de l'analyseur 606, référencé 1200 ici. L'analyseur 606 peut réaliser l'analyse par colorimétrie sur les bandelettes de test. On entend par « analyse par colorimétrie » une mesure d'absorbance ou de fluorescence sous un éclairage prédéterminé, en transmission on en réflexion. L'analyseur 606 peut alors déterminer un ou plusieurs résultats d'analyses.

L'analyseur 606 peut être un analyseur optique 1200, comprenant au moins une source de lumière 1202 (par exemple un ou plusieurs diodes électroluminescentes), 1204 et un capteur 1206 (par exemple une photodiode CCD, « *Charged Coupled Device* »*,* ou CMOS (« *Complementary Metal Oxide Semiconductor* » La source de lumière peut comprendre deux sources distinctes 1202, 1204 (par exemple deux longueurs d'onde différentes)

L'analyseur 1200 est typiquement situé de part et d'autre du logement annulaire 212, de sorte que la lumière émise par la source de lumière 1202, 1204 puisse traverser la portion cylindrique 504 transparente, puis les orifices 512 du séparateur, puis la bandelette, pour enfin atteindre le capteur optique 1206. Un séparateur optique 1208 permet de guider les deux sources de lumière 1202, 1204 pour éviter les fuites de lumière d'un chemin optique à l'autre.

Comme indiqué auparavant, l'analyseur 1200 travaille dans la zone d'analyse ZA.

Dans un mode de réalisation, l'analyseur 1200 comporte plusieurs LEDs de longueur d'onde spécifique aux différents réactifs contenus dans différentes types de bandelettes de test. Ainsi, le dispositif d'analyse d'urine peut réaliser différentes analyses avec précision.

Par exemple, chaque source de lumière 1202, 1204 peut comprendre chacune une LED blanche et une LED ultraviolette. Un séparateur vertical (non visible sur la figure 12) peut être prévu pour éviter que l'activation de la LED blanche n'excite la LED ultraviolet.

En variante, l'analyseur 1200 peut comporter une unique LED. Par exemple, la LED peut être blanche. Alors, la LED peut couvrir tout le spectre du visible. Cette configuration permet de réduire la complexité de l'analyseur.

L'analyseur 1200 peut également comporter un collimateur. Le collimateur permet d'orienter l'illumination de la ou des LEDs vers la bandelette de test.

Le capteur 1208 peut mesurer l'absorbance ou la fluorescence du réactif de la bandelette de test, notamment par transmission ou réflexion, pour établir le ou les résultats d'analyses.

Le capteur 1208 peut être surmonté d'un filtre. Le filtre permet d'augmenter la sensibilité du capteur optique à des longueurs d'ondes spécifiques. La précision d'une analyse est alors très satisfaisante.

Comme illustré en figure 12, l'analyseur 1200 est ici agencé de part et d'autre du logement annulaire 212 (et donc de part et d'autre de la portion cylindrique 504 lorsque la cartouche 202 est insérée dans la station). Une première partie du l'analyseur 1200 (par exemple la source de lumière 1202, 1204) est radialement externe au logement annulaire 212 et une deuxième partie de l'analyseur 1200 (par exemple le capteur optique 1206) est radialement interne au logement annulaire 212. L'analyseur 1200 opère par transmission de la lumière depuis la première partie vers la deuxième partie.

La première partie comprend la source de lumière 1202, 1204, par exemple sous la forme d'une paire de LEDs 1202, 1204. Lorsqu'alignés, la lumière est guidée au travers des orifices 512 pour éclairer les bandelettes de test 501. Une première LED 1202 de la paire peut être de couleur blanche pour couvrir l'ensemble du spectre visible et déterminer des changements de couleur de la bandelette de test 501. Une deuxième LED 1204 de la paire peut être monochromatique, par exemple ultraviolette, pour exciter des fluorophores et permettre l'observation de leur longueur d'onde d'émission.

La deuxième partie comprend le capteur optique 1206. Le capteur optique 1206 est ici de type spectral. Il comporte plusieurs photodiodes surmontées de filtres permettant de mesurer l'intensité de la lumière à différentes longueurs d'ondes réparties dans le visible. Le capteur 1206 est compatible avec les mesures optiques par absorbance et par fluorescence.

Le capteur 1206 peut être utilisé pour différents types de bandelettes de test. Par exemple, la bandelette de test 501 est du type immuno-chromatographique et comporte une zone de test et une zone de contrôle alignées respectivement avec les orifices 512, dont le changement de couleur permet d'obtenir un résultat. Dans un autre exemple, la bandelette de test 501 peut être une bandelette colorimétrique et comporter deux zones de test distinctes (pour tester par exemple simultanément le pH et la gravité spécifique de l'urine) alignées respectivement avec les orifices 512.

En variante, l'analyseur 1200 peut être agencé sur un moteur linéaire. De la sorte, l'analyseur peut se rapprocher d'une bandelette de test pour effectuer une analyse plus précise.

Dans un mode de réalisation, la zone d'analyse ZA de l'analyseur 1200 est confondue avec la zone d'injection ZI de l'injecteur 604. En d'autres termes, l'actionneur d'entrainement 600 n'a pas besoin d'être activé et de faire tourner la cartouche 202 entre l'injection d'urine par l'injecteur 604 et l'analyse de la bandelette 501 par l'analyseur 1200. L'injecteur 604 et l'analyseur 606, 1200 sont agencés pour travailler angulairement au même endroit et peuvent interagir avec la même bandelette (sans rotation de la cartouche 202). Les intérêts sont multiples. Un des intérêts est la cinématique : l'analyse peut commencer juste avant (par exemple au plus deux secondes ou au plus une seconde), au même moment ou juste après l'injection, de sorte que la cinétique de la réaction chimique (par exemple la vitesse de changement de couleur) de la bandelette 501 une fois au contact de l'urine peut être observé. Un autre intérêt est la précision : une bonne position pour l'injection signifie une bonne position pour l'analyse. En matière d'encombrement, comme visible sur la figure 12 le capteur optique 1206 peut être décalé, le long de l'axe de rotation A, de l'injecteur 604 (en particulier de l'aiguille 704). Il est ainsi possible d'obtenir une configuration permettant d'injecter et d'analyser une même bandelette sans mise en rotation de la cartouche (et donc sans activation de l'actionneur d'entrainement 600).

Dans un mode de réalisation, la zone d'analyse ZA de l'analyseur 1200 est confondue avec la zone de contrôle ZC du capteur de position 624. Cela permet de s'assurer que la bandelette 501 est correctement positionnée pour la mesure par l'analyseur 1200 et ainsi avoir une mesure de qualité. En particulier, le capteur optique 624 peut être l'analyseur 1200 lui-même.

Dans un mode de réalisation, la zone d'analyse ZA, la zone d'injection ZI et la zone de contrôle ZC sont confondues. Cela signifie que le capteur de position 624 détermine qu'une bandelette 501 est correctement positionnée à l'aide d'un marqueur situé dans une zone de proximité de ladite bandelette 501. Puis l'injection et l'analyse ont lieu sans que la cartouche 202 ne soit entrainée en rotation.

### Mesure directe de la position du support de test (par exemple de la bandelette).

Dans un mode de réalisation déjà mentionné plus haut, le marqueur pour le capteur de position 624 est la bandelette elle-même. De la sorte, le capteur de position 624 est configuré pour mesurer directement la position d'une bandelette par rapport au boitier 204. On s'affranchit ainsi des incertitudes de positionnement de la bandelette dans le séparateur 508 ou des incertitudes liées à la position du séparateur 508 dans le support rotatif 500.

La figure 13 illustre schématiquement des exemples d'imprécisions, à l'aide d'une vue partielle d'un séparateur 508 illustré à plat et non pas enroulé sur le support rotatif. Cinq logements 510a, 510b, 510c, 510d, 510e sont représentés, avec une bandelette respective 501a, 501b, 501c, 501d, 501e. Du fait de la souplesse du séparateur 508, lors de sa mise en place, la distance d1, d2, d3, d4 entre deux logements peut varier. Du fait de la souplesse des bandelettes et de leur insertion dans les logements, la position e1, e2, e3, e4, e5 des bandelettes dans leur logement peut varier aussi. On comprend donc qu'un marqueur positionné sur le support rotatif 500 ne permet pas de prendre en compte ces irrégularités et, ainsi ne donne la position de la bandelette à laquelle il est associé qu'à ces irrégularités près. Le capteur de position 624, lorsqu'il mesure directement la position de la bandelette, permet de s'affranchir entièrement de ces irrégularités.

En particulier, le capteur de position 624 est un capteur optique, sans contact avec la cartouche 202. Sur la figure 12, le capteur de position 624 est l'analyseur 1200 lui-même (la zone de contrôle ZC et la zone d'analyse ZA sont donc confondues). On gagne ainsi en compacité et en précision, comme indiqué précédemment.

Lorsque la cartouche 202 est en rotation et que les bandelettes 501 défilent devant le capteur de position 624, le capteur de position 624 (qui peut être l'analyseur 1200 lui-même, comme indiqué précédemment) reçoit une variation de lumière qui correspond à de la lumière passant par les orifices 512 du séparateur 508 et de part et d'autre de la bandelette 501 ou en traversant la bandelette 501. Par conséquent, le signal détecté dépend directement de la position de la bandelette 501 dans le logement annulaire 212, indépendamment de la position de la bandelette 501 par rapport au séparateur 508 ou au support rotatif 500.La commande du moteur d'entrainement 600 peut donc se faire donc indépendamment de la position du support rotatif 500 ou du séparateur 508.

Un procédé utilisant cette caractéristique va être décrit par la suite.

Le fait d'obtenir une donnée directement grâce à la bandelette permet aussi de caractériser les bandelettes. Par exemple, les bandelettes, en fonction de leur nature, peuvent présenter des extremums différents. Il est ainsi possible pour la station, en utilisant la valeur de l'extremum et en utilisant une table stockée dans la station, de savoir la nature de la bandelette présente dans la zone de contrôle (et donc dans la zone d'injection et la zone d'analyse).

### Compléments

Comme indiqué précédemment, dans un mode de réalisation, le capteur de position 624 permet de savoir si une bandelette est dans la zone de contrôle (position locale) mais pas de connaitre directement la position absolue de la bandelette dans la cartouche (en d'autres termes, de savoir quelle bandelette de la cartouche est dans la zone de contrôle ZC). Pour cela, l'ECU peut compter le nombre de bandelettes qui défilent depuis le zéro (le zéro étant l'orifice traversant 514, qui est identifiable aisément par une valeur du signal plus élevé que les autres - car la lumière ne traverse que de l'air ou des composants transparents ou quasiment transparents). En couplant le comptage avec une table stockée dans la mémoire de l'ECU, la station peut identifier la bandelette. La table peut mettre en correspondance, pour un modèle de cartouche donné, la nature de la bandelette et un numéro de la bandelette (la numérotation étant unique dans chaque cartouche). L'identification 516 permet à la station 200 d'obtenir le modèle de cartouche et donc de savoir quelle table utilisée.

Dans un mode de réalisation, le dispositif 100 comporte un capteur de présence d'urine. Le capteur de présence d'urine peut être à proximité de l'orifice de collecte. Le capteur de présence d'urine permet alors de détecter lorsque de l'urine est présente au voisinage de l'orifice de collecte. Le capteur de présence d'urine peut un capteur de température, par exemple une thermistance. Le capteur de température permet en effet de distinguer entre de l'urine et de l'eau provenant des toilettes. En outre, le capteur de température peut également être exploité pour mesurer la température de l'urine. La température de l'urine permet notamment de détecter des périodes de fécondité, par comparaison à une ou plusieurs courbes de référence. L'utilisation d'un capteur de température permet alors de réduire le nombre de composants exploités par l'ensemble de test pour réaliser une analyse. La complexité et les coûts liés à la fabrication du dispositif d'analyse d'urine sont réduits. Alternativement, le capteur de présence d'urine peut être tout type de détecteur de liquide, par exemple un capteur de type capacitif ou résistif.

La figure 14 représente une vue schématique d'un environnement d'analyse 1400 comprenant le dispositif d'analyse d'urine et de son environnement. La station 200 est contrôlée par une unité électronique de contrôle ECU 1402. L'ECU 1402 est à l'intérieur du boitier 204. L'ECU 1402 permet de contrôler les composants de l'ensemble de test pour réaliser une analyse d'urine exploitant les bandelettes de test 501 et obtenir un ou plusieurs résultats d'analyses. En particulier, l'ECU 1402 gère l'actionneur d'entrainement 600, l'actionneur de déplacement 620, le capteur de position 624, l'analyseur 1200 et la pompe 614.

L'ECU 1402 comprend typiquement un processeur 1404 et une mémoire 1406 apte à stocker des instructions que le processeur 1404 exécute. En particulier, les procédés décrits dans la description sont stockés sous forme de lignes d'instructions dans la mémoire 1406. Afin de pouvoir communiquer directement ou indirectement avec le terminal mobile 114, le serveur 116 et/ou, le cas échéant, l'activateur externe 118, la station 200 comprend un module de communication 1408, typiquement sans-fil, par exemple Bluetooth, WiFi et/ou cellulaire (GSM, 3G, 4G, 5G,4G-LTE). Une batterie 1410 permet d'alimenter en énergie les différents composants de la station 200. Pour identifier la cartouche 202 dans la station 200 (via l'identification 516), la station 200 peut comprendre un lecteur 1412, par exemple un lecteur de proximité sans contact, comme un lecteur RFID.

Le terminal mobile 114 comprend notamment un processeur 1414 et un mémoire 1416, qui permettent par exemple de faire tourner une application qui sert d'interface utilisateur pour le dispositif 100. Le serveur 116 comprend également un processeur 1418 et une mémoire 1420, pour traiter et stocker des données générées notamment par le dispositif 100.

Le dispositif 100, le terminal mobile 114 et le serveur 116 communique entre eux par un réseau de télécommunication 1422. Le réseau de télécommunication 1422 peut être hybride, en ce qui comprend un réseau WiFi ou Bluetooh et un réseau cellulaire, dont les rôles ont été expliquées précédemment.

### Procédé de pilotage

En relation avec le dispositif d'analyse d'urine 100 présentés précédemment, différents procédés vont à présent être décrit. Les étapes décrites peuvent être toutes mises en œuvre lors d'une analyse ou seulement certaines d'entre elles peuvent être mises en œuvre. En particulier, ces étapes sont exécutables à partir d'instructions stockés dans la mémoire 1305 de l'ECU 1402.

Un procédé global d'utilisation du dispositif 100 va être décrit en relation avec la figure 15.

L'étape E0 consiste à maintenir le dispositif d'analyse d'urine 100 dans une position de purge. L'ECU contrôle l'actionneur d'entrainement 600 pour mettre l'ouverture de purge 514 de la cartouche 202 dans la zone d'injection ZI (i.e. alignée avec l'injecteur 604). A cette fin, l'ECU utilise des données de position obtenues par le capteur de position 624 qui peut repérer l'ouverture 512 (par exemple par un signal lumineux plus fort car il n'y a pas de bandelette entre la source lumineuse et la photodiode). Avant la rotation de la cartouche, l'ECU a piloté l'actionneur de déplacement 620 pour mettre l'injecteur en position de retrait PR. Une fois l'ouverture de purge 514 au regard de l'injecteur, l'ECU pilote l'actionneur de déplacement 620 pour que l'injecteur 604 passe en position de purge PP. Le dispositif 100 peut ainsi être rincé de l'eau ou de résidus d'urine. Ainsi, de l'urine ou de l'eau reçu des toilettes dans le tuyau de collecte 612 peut rejoindre l'orifice de vidange 310.

Dans une étape E1 (« collecte urine »), l'ECU active la pompe 614 pour acheminer de l'urine depuis l'orifice de collecte 218 vers l'injecteur 604. L'injecteur 604 est toujours en position de purge PP et de l'urine peut ressortir par l'orifice de vidange. Cette étape permet de nettoyer et de s'assurer que l'injecteur est bien empli d'urine. L'étape E1 peut aussi comprendre une étape de pré-charge, pour amener une quantité contrôlée d'urine au niveau de l'extrémité distale 700 de l'injecteur 604

L'étape E2 (« sélection bandelette ») consiste à positionner le dispositif d'analyse d'urine 100 dans une position de sélection, en mettant la bandelette 501 désirée en regard de l'injecteur 604. L'ECU active l'actionneur de déplacement 620 pour déplacer l'injecteur 604 de la position de purge PP vers la position de retrait PR. L'ECU pilote ensuite l'actionneur d'entrainement 600 pour que la cartouche 202 soit mise en rotation et que la bandelette 501 choisie soit mise dans la zone d'injection ZI. Le choix de la bandelette 501 peut dépendre de l'analyse désirée (si différents types de bandelettes sont mis dans la cartouche 202) par l'utilisateur ou peut être automatiquement fait (bandelettes utilisées successivement selon un programme pré-établi). L'ECU récupère à nouveau les données du capteur de position 624 pour assurer que la bandelette désirée soit correctement placée. La position en absolu de la bandelette peut être connue à l'aide du capteur de position (voir plus haut et plus bas dans la description). Le positionnement exact de la bandelette est quant à lui fait grâce au capteur de position tel que décrit. Un procédé spécifique sera décrit par la suite. L'étape E2 peut prendre quelques secondes. Néanmoins, comme l'urine a déjà été collectée à l'étape E1, il n'y a pas de risque de manquer la miction de l'utilisateur.

L'étape E3 (« injection urine ») consiste par la suite à positionner le dispositif d'analyse d'urine dans une position d'injection et à injecter de l'urine. L'ECU pilote l'actionneur de déplacement 620 pour que l'injecteur passe en position d'injection PI. L'extrémité distale 700 perce alors l'opercule 511 de la cartouche 202. L'ECU pilote ensuite la pompe 614 pour injecter de l'urine sur une bandelette de test. L'urine injectée peut alors réagir avec les réactifs de la bandelette de test. Après l'injection effective, l'injecteur 604 peut être rétracté en position de retrait PR.

L'étape E4 (« mesure ») est une étape de mesure qui consiste à obtenir des données sur la bandelette désirée avec l'analyseur 606, 1200. A cette fin, l'ECU pilote l'analyseur 606, 1200 pour qu'il génère des données sur les bandelettes. L'étape E4 peut être effectuer en parallèle de l'étape E3, un peu avant l'étape E3, ou un peu après (en particulier par rapport à l'injection de la première goutte d'urine). L'obtention de données dès l'injection de la première goutte permet d'obtenir des données de cinétique de réaction. Alternativement, seules les données statiques (quand la réaction des bandelettes est stabilisée), sont utilisées. Lorsque la zone d'analyse ZA et la zone d'injection ZI du logement annulaire sont confondus (i.e. lorsque l'analyseur 606, 1200 est positionné radialement au même endroit que l'injecteur 604), l'ECU n'a pas besoin de faire tourner la cartouche 202.

L'étape E5 (« purge ») consiste à purger le dispositif d'analyse d'urine. L'ECU pilote l'actionneur d'entrainement 600 pour aligner l'orifice de purge 514 avec l'injecteur 604, puis pilote l'actionneur de déplacement 620 pour mettre l'injecteur en position de purge PP, puis active la pompe 614 pour pousser de l'air. Alors, l'urine est expulsée du dispositif d'analyse d'urine via le canal de purge et par l'orifice de vidange 310. Le dispositif d'analyse d'urine 100 est ainsi à la même position qu'en étape E0. Au préalable de l'alignement de l'orifice de purge 514 avec l'injecteur 604, l'injecteur 604 peut être remis en position de retrait PR en pilotant l'actionneur de déplacement 620, si cela n'a pas été fait à la fin de l'étape E3.

L'activation de l'étape E1 peut impliquer que l'ECU reçoive une demande d'analyse d'urine. La demande d'analyse peut provenir de l'activateur déportée. La demande d'analyse peut également être commandée depuis le smartphone 114. La demande d'analyse peut également être réalisée automatiquement, lorsque l'utilisateur est à proximité des toilettes.

De la même façon, l'activation de l'étape E1 peut impliquer que l'ECU détecte de l'urine à proximité de l'orifice de collecte 218. Si aucun jet d'urine n'est reçu dans un délai imparti, alors le dispositif d'analyse d'urine retourne à l'étape E0. Si, au contraire, un jet d'urine est détecté, l'étape E1 est mise en œuvre.

L'injection d'urine à l'étape E2 peut se faire en deux temps. Par exemple, la seringue automatisée 80 peut injecter deux fois entre 2,5 microlitres et 3,5 microlitres. Cette solution permet de prendre en compte un temps de réaction et de migration de l'urine sur la bandelette de test 501.

L'analyseur réalise une analyse par colorimétrie sur la bandelette de test. Le dispositif d'analyse d'urine en déduit le ou les résultats d'analyses. L'analyse réalisée dépend du type de bandelette de test. L'analyse réalisée peut également dépendre d'un choix de l'utilisateur. L'analyse effectuée peut également être choisie en fonction de l'utilisateur identifié.

Une étape E6 de traitement des données et de transmission du ou des résultats peut être mise en œuvre. L'ECU traite les données reçues par l'analyseur 606 et instruit un émetteur de transmettre le ou les résultats, par exemple directement au terminal mobile 114 de l'utilisateur. Le ou les résultats peuvent également être envoyés vers le serveur 116. L'utilisateur peut par exemple visualiser et exploiter le ou les résultats sur l'application du smartphone 114, ou sur un site web. Le ou les résultats peuvent également être envoyés vers un professionnel de santé. L'étape E6 peut être effectué à n'importe quel moment après l'étape E4.

Il est à noter que les étapes E2 à E4 peuvent être répétées plusieurs fois. Ainsi, plusieurs bandelettes de test reçoivent de l'urine et sont analysées. Alors, plusieurs analyses peuvent être réalisées à partir d'une unique aspiration d'urine à l'étape E1.

L'étape E0 consiste à maintenir le dispositif d'analyse d'urine dans la position de purge, en mettant l'injecteur 604 en position de purge PP.

La mise en position de la bandelette va être à présent décrit avec plus de détails. Comme indiqué auparavant, le capteur de position 624 peut recevoir un signal (par exemple un signal lumineux) dont l'intensité varie en fonction des éléments qui défilent devant ledit capteur de position 624. En particulier, le signal atteint un extremum lorsqu'une bandelette se trouve dans la zone de contrôle ZC, c'est-à-dire en regard radialement du capteur de position 624. Dans le cas d'un moteur 600 pas-à-pas, une valeur de signal est déterminée pour chaque pas. Le graphe 1600 en figure 16 présentent trois courbes : le signal 1602 obtenu par le capteur de position 624, le signal filtré 1604 (typiquement une moyenne de plusieurs valeurs) et la valeur 1606 du dernier extremum. L'ordonnée est une unité d'intensité du signal optique et l'abscisse est un nombre de pas du moteur d'entrainement 600. Les intensités les plus fortes correspondent à un alignement des orifices 512 du séparateur 508 avec le capteur de position 624 et les intensités les plus faibles correspondent au séparateur 508 qui bloque la lumière.

Dans une étape F1, l'ECU met en mouvement les bandelettes 501 dans un sens donné, qui défilent devant le capteur de position 624. Par exemple, l'ECU peut piloter le moteur 600 dans un sens. Les bandelettes 501 peuvent défiler devant le capteur de position 624 selon deux sens opposés. Un sens est choisi à l'étape F1.

Dans une étape F2, généralement concomitante à l'étape F1, l'ECU reçoit et analyse l'évolution du signal de retour durant le défilement. Une mesure peut être obtenue à chaque pas du moteur d'entrainement 600 ou à intervalle de temps régulier (par exemple toutes les 0,1 seconde), par exemple si moteur est à déplacement continu.

Dans une étape F3, l'ECU identifie un extremum local du signal. Cela signifie que l'ECU a détecté que la valeur du signal s'est mise à diminuer après un pic (au signe près). A cet égard, l'ECU a besoin de détecter au moins trois valeurs de signal, correspondant à trois positions de la cartouche différente, pour lequel la valeur intermédiaire est la plus élevée (au signe près) des trois valeurs. Ces trois valeurs sont détectables sur trois pas successifs par exemple.

Plus génériquement (dans le cas d'un moteur pas à pas), si on suppose qu'il y a un extremum au niveau du pas N (instant t1, ou t2 pour le signal filtré, sur la figure 16), l'ECU observe une fenêtre de ± k mesures (donc 2xk +1pas), et si valeur[N] = max(valeur[N-k;N+k]), l'ECU détermine que valeur[N] est un extremum (instant t3 sur la figure 16). Néanmoins, la cartouche est alors en position N+k, c'est à dire que l'extremum de la position N a été dépassé de k pas à ce moment-là. Une solution est donc de revenir en arrière de k pas. En revanche, comme la chaine d'entrainement présente du backlash, l'ECU ne peut pas simplement compter k pas dans l'autre sens : l'ECU cherche à retrouver la valeur de l'extremum (modulo une marge), ce qui signifie que la cartouche est dans la position désirée. Les étapes F4 et F5 décrivent cela en détail.

Dans une étape F4, en réponse à l'étape F3, l'ECU met en mouvement les bandelettes dans l'autre sens à celui du sens de l'étape F1. Par exemple, l'ECU peut piloter le moteur 600 dans l'autre sens ou, provoquer une inversion du sens de rotation dans la chaine d'engrenage 608 (en débrayant ou embrayant un engrenage).

Dans une étape F5, l'ECU met en position la bandelette qui a généré l'extremum. En d'autres termes, l'ECU arrête l'actionneur d'entrainement 600 pour mettre la bandelette à l'endroit désiré, qui est ici la zone d'injection ZI ou la zone d'analyse ZA (qui peuvent être confondues, comme expliqué précédemment). En particulier, lorsque la zone de contrôle ZC est confondu avec la zone d'injection ZI et/ou la zone d'analyse ZA, l'endroit désiré est la zone de contrôle ZC, c'est-à-dire la zone en regard du capteur de position 624. L'étape F5 peut comprendre une sous-étape F51 d'identification par l'ECU d'une valeur du signal proche de la valeur du premier extremum local et une sous-étape F52 d'arrêt du défilement dans l'autre sens. Par « proche » il est entendu une valeur identique ou similaire à un écart prédéterminé près (définie par exemple lors de tests). L'identification d'une valeur proche permet d'arrêter le défilement au niveau de la position correspondant exactement à l'extremum. Si l'ECU devait déterminer un nouvel extremum, il aurait fallu que le défilement inverse aille au-delà de la position correspondant à l'extremum originel.

En défilement inverse (étape F4), c'est-à-dire lorsque l'ECU cherche à retrouver la position de la cartouche correspond à l'extremum, le défilement peut se faire à la même vitesse que dans le sens de l'étape F1, afin d'avoir les mêmes conditions d'acquisition qu'aux étapes F1 et F2. Pour cela, le déplacement pas à pas est particulièrement adapté.

Lorsque l'ECU détecte un extremum, la cartouche 202 a été tournée de quelques degrés seulement, de sorte qu'aucune autre bandelette n'a encore défilé devant le capteur de position 624 ou, à tout le moins aucun autre extremum ne peut être identifié.

Un tel procédé de mise en position présente plusieurs avantages : premièrement, la mesure de la position de la bandelette directement permet de s'affranchir des imprécisions du backlash dans la chaine mécanique (par exemple le jeu entre les pignons de train d'engrenages 608 lorsqu'il change de sens de rotation) et de la position de la bandelette (voir figure 13). De plus, l'identification d'un extremum permet de s'affranchir des variations des valeurs du signal entre bandelettes. Comme illustré sur le graphe 1600, les valeurs des extrema peuvent variés. Deux extremums successifs (associés à deux bandelettes successives) peuvent ainsi avoir des valeurs différentes sans que le procédé ne soit mis en échec. Les écarts d'extrema peuvent venir des irrégularités de positionnements des bandelettes ou de la nature même des bandelettes (une cartouche 202 peut intégrer différents types de bandelettes, qui ne présentent pas la même réponse au capteur optique, tout autre paramètre étant égal par ailleurs). Deuxièmement, le procédé permet de gérer le temps de latence de traitement des données par l'électronique (capteur de position et ECU) : le fait que le pic ne soit pas connu en temps réel est compensé par le déplacement en sens inverse. L'électronique embarqué peut donc être moins gourmand en énergie, moins cher et plus robuste.

On remarque ici que ce procédé peut être mis en oeuvre indépendamment de la forme annulaire du logement 212. Toutefois, le mouvement de rotation de la cartouche 202 dans la station 200 est particulièrement adaptée à ce genre de procédé, et ce pour toutes les bandelettes de la cartouche (du fait de la symétrie de révolution).

En référence au comptage des bandelettes pour identifier la bandelette qui est dans la zone de contrôle, l'ECU peut compter les extremums que le capteur de position a vu défiler. Par exemple, si l'ECU détermine qu'il faut utiliser la bandelette numéro 54 (sur 90 par exemple), l'ECU peut compter 90 extremums depuis le zéro.

### Complément sur les supports de test

Les supports de test comprennent un réactif qui réagit au contact de l'urine. Dans un mode de réalisation, le réactif est un réactif sec. En particulier, les supports de test sont des bandelettes de test, qui vont être décrites plus en détail ci-dessous.

Les bandelettes de test 501 peuvent être du type immunodosage à flux latéral ou vertical, en anglais « *lateral or vertical flow immunoassay* ». Alors, les bandelettes de test 501 comportent un tampon d'échantillonnage et un tampon d'absorption 02. Une membrane de nitrocellulose s'étend entre le tampon d'échantillonnage et le tampon d'absorption. Alors, lorsqu'un échantillon d'urine est introduit sur le tampon d'échantillonnage, il migre par capillarité jusqu'au tampon d'absorption en passant au travers d'un tampon conjugué, d'une ou plusieurs lignes de test, et d'une ligne de contrôle. Le tampon conjugué, le ou les lignes de test et la ligne de contrôle contiennent des réactifs.

Le tampon conjugué comporte notamment des anticorps de détection sensibles à des composés contenus dans l'urine. Si les composés sont présents lorsque l'échantillon d'urine traverse le tampon conjugué, alors les anticorps se fixent aux composés pour former des marqueurs. Les marqueurs migrent vers une ligne de test. La ligne de test comporte notamment des anticorps de test. Les anticorps de test se lient avec les marqueurs et les retiennent sur la ligne de test. Alors, une ligne colorée se forme et la densité de la ligne varie en fonction de la concentration de marqueurs présents. L'échantillon restant migre vers une ligne de contrôle. La ligne de contrôle comporte des anticorps de contrôle, permettant d'indiquer que l'échantillon a traversé la membrane de nitrocellulose.

Par exemple, les bandelettes de test peuvent être des bandelettes de type ELISA. Ce type de bandelette de test permet une détection de l'hormone de grossesse hCG dans l'urine. Alors, l'anticorps de détection peut être « mouse monoclonal beta hCG », l'anticorps de test peut être « goat poluclonal anti-mouse IgG » et l'anticorps de contrôle peut être « rabbit polyclonal anti-mouse igG ».

Les bandelettes de test peuvent être de type bandelettes colorimétriques classiques. Alors, chaque bandelette de test comporte au moins un tampon contenant un ou plusieurs réactifs sensibles à un ou plusieurs composés contenus dans l'échantillon d'urine. Par exemple, le ou les composés peuvent être : l'hormone LH, l'hormone HCG, les leucocytes/nitrites, les urobilinogène/bilirubine, les protéines, le pH, la gravité spécifique et/ou le glucose.

D'autres types de réactions peuvent utiliser réactifs ou composés conçus pour détecter la présence d'un analyte particulier (par exemple des polymères à empreinte moléculaire "MIP" ou "Molecularly imprinted polymers"), notamment un principe actif de médicament ou un métabolite de principe actif de médicament dans l'urine. Dans ce cas, le dispositif peut être utilisé pour contrôler l'observance d'un utilisateur pour un traitement médicamenteux, notamment vérifier qu'il prend bien son traitement ou l'alerter lorsqu'il a omis de le prendre.

Chaque bandelette de tests est globalement rectangulaire. Une largeur de chaque bandelette de test peut être comprise entre 0,5 mm et 3 mm, par exemple environ 1 mm. Une longueur de chaque bandelette de test peut être comprise entre 10 et 15 mm, par exemple 12 mm ou environ 12 mm. Alternativement, chaque bandelette de test peut avoir une forme quelconque, par exemple carrée ou circulaire. La forme et les dimensions des bandelettes de test permettent de stocker un nombre important de bandelettes de test dans le dispositif d'analyse d'urine (au moins 50 bandelettes, voire au moins 100 bandelettes). En effet, il apparait même possible de stocker jusqu'à 120 bandelettes de test, ce qui correspond à 4 mois d'analyses lorsqu'un utilisateur réalise une analyse par jour.

Le ou les résultats d'analyses peuvent être une ou plusieurs grandeur(s) révélant l'un(e) parmi : une période de fécondité, une grossesse, des infections urinaires, des problèmes de foie, des insuffisances rénales, une lithiase urique, une déshydratation, une maladie cardiaque et/ou un diabète. Le ou les résultats peuvent également être un indicateur de l'observance médicamenteuse.

L'invention est définie par les revendications jointes.

## Revendications

1. Station (200) pour dispositif d'analyse d'urine (100), la station comprenant :
- un boitier (204), destiné à être positionné à l'intérieur d'une cuvette de toilettes (102),
- un logement annulaire (212), autour d'un axe de rotation (A), dans le boitier (204), le logement annulaire (212) étant configuré pour recevoir au moins partiellement une cartouche (202) montée à rotation autour de l'axe de rotation (A) dans la station (200) et comprenant une pluralité de supports de test (501),
- un injecteur (604), positionné dans le boitier (204) et radialement interne au logement annulaire (212), l'injecteur étant configuré pour injecter un volume contrôlé d'urine sur au moins un support de test, l'injecteur étant monté mobile en translation par rapport au boitier,
dans laquelle l'injecteur comprend une extrémité distale d'injection dont le déplacement en translation se fait selon une direction radiale ou sensiblement radiale par rapport à l'axe de rotation.

2. Station selon la revendication 1, dans laquelle :
- l'injecteur (604) est entrainé en translation au niveau d'un premier axe (T1),
- l'extrémité distale (700) d'injection se déplace en translation selon un deuxième axe (T2), dit axe d'injection, et le premier axe de translation (T1) n'est pas confondu avec le deuxième axe (T2), lorsque ces derniers sont projetés dans un plan orthogonal à l'axe de rotation (A).

3. Station selon l'une quelconque des revendications 1 à 2, comprenant un coupleur mécanique (602), sur l'axe de rotation (A), apte à coopérer avec la cartouche (202) pour la mise en rotation de cette dernière.

4. Station selon la revendication 3, dans laquelle le deuxième axe de translation (T2) traverse ledit coupleur mécanique (602) et le premier axe de translation (T1) est déporté par rapport au coupleur mécanique (602).

5. Station selon la revendication 3 ou 4, dans laquelle dans laquelle l'injecteur (604) est courbé et la forme courbée de l'injecteur contourne le coupleur mécanique.

6. Station selon l'une quelconque des revendications 1 à 5, dans laquelle « sensiblement radiale » signifie moins de 5° de part et d'autre de la direction radiale, voire moins de 2°.

7. Station selon l'une quelconque des revendications 1 à 6, dans laquelle :
- l'injecteur comprend une extrémité proximale (702), opposée à l'extrémité distale (700), et configuré pour recevoir de l'urine,
- l'extrémité distale (700) et l'extrémité proximale (702) sont reliées entre elles par une portion intermédiaire (710) comprenant deux virages successifs inversés (706, 708),
- et, par exemple, le virage (706) le plus proche de l'extrémité distal (700) a un rayon de courbure plus faible que le virage (708) le plus proche de l'extrémité proximale (708).

8. Station selon l'une quelconque des revendications 1 à 7, dans laquelle l'injecteur (604) est rigide.

9. Station selon l'une quelconque des revendications 1 à 8, comprenant en outre un actionneur de déplacement (620), configuré pour déplacer l'injecteur (604) en translation, dans laquelle l'injecteur est par exemple solidaire d'un écrou (800) apte à recevoir une tige filetée entrainée en rotation par l'actionneur de déplacement (620).

10. Station selon l'une quelconque des revendications 1 à 9, dans laquelle l'injecteur (604) est monté sur un chariot (900), dans laquelle le chariot reprend les efforts transmis par l'actionneur de déplacement (620).

11. Station selon l'une quelconque des revendications 1 à 10 :
- dans laquelle le boitier comprend un collecteur d'urine, ledit collecteur étant fluidiquement relié à l'injecteur,
- comprenant une unité électronique de contrôle, l'unité électronique de contrôle (1402) étant dans le boitier (204),
- comprenant en outre un analyseur (1200), positionné au moins partiellement radialement à l'extérieur du logement annulaire et configuré pour analyser optiquement au moins un support de test,
- l'analyseur est positionné en regard de l'extrémité distale de l'injecteur, lorsqu'ils sont projetés dans plan orthogonal à l'axe de rotation.

12. Station (200) selon l'une quelconque des revendications 1 à 11, dans lequel le boitier (204) a un diamètre, mesuré dans la direction normale à l'axe de rotation (A), compris entre 50 mm et 150 mm.

13. Station (200) selon l'une quelconque des revendications 1 à 12, dans lequel le boitier (204) est configuré pour être agencé dans la cuvette (106) des toilettes de manière amovible.

14. Dispositif d'analyse d'urine (100) comprenant une station (200) selon l'une quelconque des revendications précédentes et une cartouche (202), la cartouche étant configurée pour être au moins partiellement reçue dans le logement annulaire du boitier.

15. Dispositif d'analyse d'urine selon la revendication 14, dans lequel la cartouche (202) comprend un support rotatif (500) configuré pour être entrainé en rotation par la station (200), la cartouche (202) comprenant des bandelettes de test (501) attachées et solidaires du dans le support rotatif (500), les bandelettes (501) étant disposées parallèlement entre elles.

## Patentansprüche

1. Station (200) für eine Urinanalysevorrichtung (100), wobei die Station umfasst :
- ein Gehäuse (204), das dazu bestimmt ist, innerhalb eines Toilettenbeckens (102) positioniert zu werden,
- ein ringförmiges Gehäuse (212) um eine Drehachse (A) in dem Gehäuse (204), wobei das ringförmige Gehäuse (212) so konfiguriert ist, dass es zumindest teilweise eine Patrone (202) aufnimmt, die drehbar um die Drehachse (A) in der Station (200) angebracht ist und eine Vielzahl von Testträgern (501) umfasst,
- einen Injektor (604), der in dem Gehäuse (204) positioniert ist und radial innerhalb der ringförmigen Aufnahme (212) liegt, wobei der Injektor so konfiguriert ist, dass er ein kontrolliertes Volumen an Urin auf mindestens einen Testträger injiziert, wobei der Injektor translationsbeweglich in Bezug auf das Gehäuse montiert ist,
wobei der Injektor ein distales Injektionsende umfasst, dessen Translationsbewegung in einer radialen oder im Wesentlichen radialen Richtung in Bezug auf die Drehachse erfolgt.

2. Station nach Anspruch 1, wobei :
- der Injektor (604) um eine erste Achse (T1) translatorisch angetrieben wird,
- das distale Injektionsende (700) translatorisch entlang einer zweiten Achse (T2), der sogenannten Injektionsachse, bewegt wird und die erste Translationsachse (T1) nicht mit der zweiten Achse (T2) zusammenfällt, wenn diese in eine Ebene projiziert werden, die orthogonal zur Rotationsachse (A) verläuft.

3. Station nach einem der Ansprüche 1 bis 2, mit einer mechanischen Kupplung (602) auf der Drehachse (A), die mit der Kartusche (202) zusammenwirken kann, um diese in Drehung zu versetzen.

4. Station nach Anspruch 3, bei der die zweite Translationsachse (T2) durch die mechanische Kupplung (602) verläuft und die erste Translationsachse (T1) gegenüber der mechanischen Kupplung (602) versetzt ist.

5. Station nach Anspruch 3 oder 4, wobei in der Station die Einspritzdüse (604) gekrümmt ist und die gekrümmte Form der Einspritzdüse den mechanischen Koppler umgeht.

6. Station nach einem der Ansprüche 1 bis 5, wobei "im Wesentlichen radial" weniger als 5° auf jeder Seite der radialen Richtung oder sogar weniger als 2° bedeutet.

7. Station nach einem der Ansprüche 1 bis 6, wobei :
- der Injektor ein proximales Ende (702) umfasst, das dem distalen Ende (700) gegenüberliegt und so konfiguriert ist, dass es Urin aufnimmt,
- das distale Ende (700) und das proximale Ende (702) durch einen Zwischenabschnitt (710) miteinander verbunden sind, der zwei aufeinanderfolgende, umgekehrte Kurven (706, 708) umfasst,
- und z. B. die Kurve (706), die dem distalen Ende (700) am nächsten liegt, einen kleineren Krümmungsradius hat als die Kurve (708), die dem proximalen Ende (708) am nächsten liegt.

8. Station nach einem der Ansprüche 1 bis 7, wobei der Injektor (604) starr ist.

9. Station nach einem der Ansprüche 1 bis 8, die außerdem einen Verschiebungsaktuator (620) umfasst, der so konfiguriert ist, dass er die Düse (604) translatorisch verschiebt, wobei die Düse beispielsweise fest mit einer Mutter (800) verbunden ist, die eine Gewindestange aufnehmen kann, die von dem Verschiebungsaktuator (620) in Drehung versetzt wird.

10. Station nach einem der Ansprüche 1 bis 9, bei der der Injektor (604) auf einem Schlitten (900) montiert ist, wobei der Schlitten die von dem Verschiebungsaktuator (620) übertragenen Kräfte übernimmt.

11. Station nach einem der Ansprüche 1 bis 10 :
- wobei das Gehäuse einen Urinsammler umfasst, wobei der Sammler fluidisch mit dem Injektor verbunden ist,
- eine elektronische Steuereinheit umfasst, wobei sich die elektronische Steuereinheit (1402) in dem Gehäuse (204) befindet,
- ferner umfassend einen Analysator (1200), der zumindest teilweise radial außerhalb des ringförmigen Gehäuses positioniert und so konfiguriert ist, dass er mindestens einen Testträger optisch analysiert,
- Der Analysator ist gegenüber dem distalen Ende des Injektors positioniert, wenn sie in einer Ebene orthogonal zur Rotationsachse projiziert werden.

12. Station (200) nach einem der Ansprüche 1 bis 11, wobei das Gehäuse (204) einen Durchmesser, gemessen in der Richtung senkrecht zur Drehachse (A), zwischen 50 mm und 150 mm hat.

13. Station (200) nach einem der Ansprüche 1 bis 12, wobei das Gehäuse (204) so gestaltet ist, dass es herausnehmbar in der Toilettenschüssel (106) angeordnet ist.

14. Urinanalysevorrichtung (100) mit einer Station (200) nach einem der vorhergehenden Ansprüche und einer Kartusche (202), wobei die Kartusche so konfiguriert ist, dass sie zumindest teilweise in der ringförmigen Aufnahme des Gehäuses aufgenommen werden kann.

15. Urinanalysevorrichtung nach Anspruch 14, wobei die Kartusche (202) eine drehbare Halterung (500) umfasst, die so konfiguriert ist, dass sie von der Station (200) in Drehung versetzt wird, wobei die Kartusche (202) Teststreifen (501) umfasst, die an der drehbaren Halterung (500) befestigt und mit dieser einstückig sind, wobei die Streifen (501) parallel zueinander angeordnet sind.

## Claims

1. A station (200) for a urine analysis device (100), the station comprising :
- a case (204), intended to be positioned inside a toilet bowl (102),
- an annular housing (212), about an axis of rotation (A), in the case (204), the annular housing (212) being configured to at least partially receive a cartridge (202) mounted for rotation about the axis of rotation (A) in the station (200) and comprising a plurality of test holders (501),
- an injector (604), positioned in the case (204) and radially internal to the annular housing (212), the injector being configured to inject a controlled volume of urine onto at least one test support, the injector being mounted for translational movement relative to the case,
wherein the injector comprises a distal injection end whose translational displacement is in a radial or substantially radial direction relative to the axis of rotation.

2. The station according to claim 1, wherein :
- the injector (604) is driven in translation about a first axis (T1),
- the distal injection end (700) moves in translation along a second axis (T2), known as the injection axis, and the first translation axis (T1) is not coincident with the second axis (T2), when these are projected in a plane orthogonal to the axis of rotation (A).

3. The station according to any one of claims 1 to 2, comprising a mechanical coupler (602), on the axis of rotation (A), able to cooperate with the cartridge (202) for rotating the latter.

4. The station according to claim 3, wherein the second translation axis (T2) passes through said mechanical coupler (602) and the first translation axis (T1) is offset with respect to the mechanical coupler (602).

5. The station according to claim 3 or 4, wherein the injector (604) is curved and the curved shape of the injector bypasses the mechanical coupler.

6. The station according to any one of claims 1 to 5, wherein "substantially radial" means less than 5° either side of the radial direction, or even less than 2°.

7. The station according to any one of claims 1 to 6, wherein :
- the injector comprises a proximal end (702), opposite the distal end (700), and configured to receive urine,
- the distal end (700) and the proximal end (702) are interconnected by an intermediate portion (710) comprising two successive inverted turns (706, 708),
- and, for example, the bend (706) closest to the distal end (700) has a smaller radius of curvature than the bend (708) closest to the proximal end (708).

8. The station according to any one of claims 1 to 7, wherein the injector (604) is rigid.

9. The station according to any one of claims 1 to 8, further comprising a displacement actuator (620), configured to displace the injector (604) in translation, wherein the injector is for example integral with a nut (800) adapted to receive a threaded rod driven in rotation by the displacement actuator (620).

10. The station according to any one of claims 1 to 9, wherein the injector (604) is mounted on a carriage (900), wherein the carriage takes up the forces transmitted by the displacement actuator (620).

11. The station according to any one of claims 1 to 10:
- wherein the case comprises a urine collector, said collector being fluidly connected to the injector,
- comprising an electronic control unit, the electronic control unit (1402) being in the case (204),
- further comprising an analyzer (1200), positioned at least partially radially outside the annular housing and configured to optically analyze at least one test support,
- the analyzer is positioned opposite the distal end of the injector, when projected in a plane orthogonal to the axis of rotation.

12. The station (200) according to any one of claims 1 to 11, wherein the case (204) has a diameter, measured in the direction normal to the axis of rotation (A), of between 50 mm and 150 mm.

13. The station (200) according to any one of claims 1 to 12, wherein the case (204) is configured to be removably arranged in the toilet bowl (106).

14. A urine analysis device (100) comprising a station (200) according to any of the preceding claims and a cartridge (202), the cartridge being configured to be at least partially received in the annular housing of the case.

15. A urine analysis device according to claim 14, wherein the cartridge (202) comprises a rotatable holder (500) configured to be rotated by the station (200), the cartridge (202) comprising test strips (501) attached to and integral with the rotatable holder (500), the strips (501) being arranged parallel to one another.
